# EUROPEAN PATENT APPLICATION

(11) **EP 1 338 652 A2**
(43) Date of publication of application: **27.08.2003**
(21) Application number: 03011698.2
(22) Date of filing: 19.03.1999
(51) Int. Cl.: C12N 15/29, C07K 14/415, C12N 5/10, C12Q 1/68

(54) **Plant cell cyclin genes**

(30) Priority: 23.03.1998 US 78948 P
(62) Divisional of application: 99912723.6
(71) Applicant: E.I.Du Pont de Nemours and Company, Wilmington, Deleware 19898 (US)
(72) Inventor: The designation of the inventor has not yet been filed
(74) Representative: Webber, Philip Michael

(57) **Abstract**

This invention relates to an isolated nucleic acid fragment encoding a cyclin protein. The invention also relates to the construction of a chimeric gene encoding all or a portion of the cyclin protein, in sense or antisense orientation, wherein expression of the chimeric gene results in production of altered levels of the cyclin protein in a transformed host cell.

SEQUENCE LISTING

<110> E. I. DU PONT DE NEMOURS AND COMPANY

<120> PLANT CELL CYCLIN GENES

<130> BB-1149

<150> 60/078,735
<151> 1998 March 20

<160> 32

<170> MICROSOFT OFFICE 97

<210> 1
<211> 1071
<212> DNA
<213> Zea mays

<400> 1
ccggaattcc cggggtcgac ccacgcgtcc ggccgcgcgc cgtggcgccc gccgacctcc 60
agctctccgg gtcctacgcc tccgacatct acacctacct ccgctccctg gaggtggatc 120
cgcagcggcg gtccagatcc gattacatcg aggcggtgca ggcggacgtc acggcccaca 180
tgcggagcat cctcgtcgac tggctcgtcg aggtcgccga ggagtacaag ctcgtcgcgg 240
acacgctcta cctcaccatc tcttatgtcg accgcttcct ctccgtcaac gcgctcggcc 300
gtgacaagct gcagctcctt ggcgttgcct ccatgctcat tgccgcgaag ttcgaggaga 360
tcagcccgcc gcacccggag gacttctgct acatcacaga caacacctac accaaagagg 420
agctcctcaa gatggagagc gacatactca agcttctcaa gttcgagttg ggcaatccta 480
caatcaagac cttcctgaga cgtttcataa gatctgccca tgaagacaag aagggctcca 540
tcttgttaat ggaattcttg ggagctacct tcgctgagct gagtctacta gattatggct 600
gcctccggtt cttgccatca gtagttgctg cttcagtcat gtttgttgct aggcctgaca 660
ttgatccaaa taccaatccg tggaacacaa agctgcagaa gatgactggc tacaaagttt 720
ctgaactcaa ggattgcatc gtagccatac atgacttgca gctcaacagg aaatgtccat 780
cattaacggc aattcgagac aagtacaagc agcacaagtt caaatgcgtg tcattgatcc 840
tcgtgcctgt cgtgatccct acttcatact ttgaagactt agctgagtag ctgctctcgg 900
actgtaccgc tgtaaggcta acaatctgag ctctccttga gctcttaggg acaagcagaa 960
aataaccgtt tgatgagctt tcctctcatt taagtagcgt ggtgaaagct atttgtttga 1020
ggttctttag gattaaaaaa aaaaaaaaaa aaaaaaaaaa aaaaaaaaaa a 1071

<210> 2
<211> 295
<212> PRT
<213> Zea mays

<400> 2
Gly Ile Pro Gly Val Asp Pro Arg Val Arg Pro Arg Ala Val Ala Pro
1               5                   10                  15

Ala Asp Leu Gln Leu Ser Gly Ser Tyr Ala Ser Asp Ile Tyr Thr Tyr
            20                  25                  30

Leu Arg Ser Leu Glu Val Asp Pro Gln Arg Arg Ser Arg Ser Asp Tyr
        35                  40                  45

Ile Glu Ala Val Gln Ala Asp Val Thr Ala His Met Arg Ser Ile Leu
    50                  55                  60

Val Asp Trp Leu Val Glu Val Ala Glu Glu Tyr Lys Leu Val Ala Asp
65                  70                  75                  80

Thr Leu Tyr Leu Thr Ile Ser Tyr Val Asp Arg Phe Leu Ser Val Asn
                85                  90                  95

Ala Leu Gly Arg Asp Lys Leu Gln Leu Leu Gly Val Ala Ser Met Leu
            100                 105                 110

Ile Ala Ala Lys Phe Glu Glu Ile Ser Pro Pro His Pro Glu Asp Phe
        115                 120                 125

Cys Tyr Ile Thr Asp Asn Thr Tyr Thr Lys Glu Glu Leu Leu Lys Met
    130                 135                 140

Glu Ser Asp Ile Leu Lys Leu Leu Lys Phe Glu Leu Gly Asn Pro Thr
145                 150                 155                 160

Ile Lys Thr Phe Leu Arg Arg Phe Ile Arg Ser Ala His Glu Asp Lys
                165                 170                 175

Lys Gly Ser Ile Leu Leu Met Glu Phe Leu Gly Ser Tyr Leu Ala Glu
            180                 185                 190

Leu Ser Leu Leu Asp Tyr Gly Cys Leu Arg Phe Leu Pro Ser Val Val
        195                 200                 205

Ala Ala Ser Val Met Phe Val Ala Arg Pro Asp Ile Asp Pro Asn Thr
    210                 215                 220

Asn Pro Trp Asn Thr Lys Leu Gln Lys Met Thr Gly Tyr Lys Val Ser
225                 230                 235                 240

Glu Leu Lys Asp Cys Ile Val Ala Ile His Asp Leu Gln Leu Asn Arg
                245                 250                 255

Lys Cys Pro Ser Leu Thr Ala Ile Arg Asp Lys Tyr Lys Gln His Lys
            260                 265                 270

Phe Lys Cys Val Ser Leu Ile Leu Val Pro Val Val Ile Pro Thr Ser
        275                 280                 285

Tyr Phe Glu Asp Leu Ala Glu
    290                 295

<210>  3
<211>  435
<212>  DNA
<213>  Glycine max

<220>
<221>  unsure
<222>  (1)

<220>
<221>  unsure
<222>  (86)

<220>
<221>  unsure
<222>  (88)

<220>
<221>  unsure
<222>  (216)..(217)

<220>
<221>  unsure
<222>  (231)

<220>
<221>  unsure
<222>  (240)

<220>
<221> unsure
<222> (307)

<220>
<221> unsure
<222> (372)

<220>
<221> unsure
<222> (377)

<220>
<221> unsure
<222> (427)

<400> 3
nactccatct tcttccatcc atttcctctt tctcgatctg ttccaaattc acttcacaca 60
caggaaagaa gatggagact cgcgcngncg caaagagaaa ggcaaatgcc gccaccatag 120
tctttgtcga aaaacaatac cccaacaaga ggcagcgggt tgtgttgggt gaacttccca 180
atttacaaaa ccttattgtc tccgaaactc aaaatnngcg caaagagaag ntcctatgtn 240
ggaagaatcc caatgagaag aaaccatcac ccacaaacaa caacaccttt ccttcccctc 300
agatcancga atcttatgat tcggatatcc acgggtatct tcgtgaaatg gagatgcaga 360
ataagagaag ancaatngtt gatacattga aaaggttaga aaatcgttac ccaaccatgg 420
agcaatntgg tgatt 435

<210> 4
<211> 110
<212> PRT
<213> Glycine max

<220>
<221> UNSURE
<222> (6)

<220>
<221> UNSURE
<222> (49)

<220>
<221> UNSURE
<222> (54)

<220>
<221> UNSURE
<222> (57)

<220>
<221> UNSURE
<222> (79)

<220>
<221> UNSURE
<222> (101)..(102)

<400> 4
Met Glu Thr Arg Ala Xaa Ala Lys Arg Lys Ala Asn Ala Ala Thr Ile
1               5                   10                  15

Val Phe Val Glu Lys Gln Tyr Pro Asn Lys Arg Gln Arg Val Val Leu
            20                  25                  30

Gly Glu Leu Pro Asn Leu Gln Asn Leu Ile Val Ser Glu Thr Gln Asn
        35                  40                  45

Xaa Arg Lys Glu Lys Xaa Leu Cys Xaa Lys Asn Pro Asn Glu Lys Lys
    50                  55                  60

Pro Ser Pro Thr Asn Asn Asn Thr Phe Pro Ser Pro Gln Ile Xaa Glu
65                  70                  75                  80

Ser Tyr Asp Ser Asp Ile His Gly Tyr Leu Arg Glu Met Glu Met Gln
                85                  90                  95

Asn Lys Arg Arg Xaa Xaa Val Asp Thr Leu Lys Arg Leu Glu
            100                 105                 110

<210>  5
<211>  847
<212>  DNA
<213>  Triticum aestivum

<220>
<221>  unsure
<222>  (584)

<220>
<221>  unsure
<222>  (686)

<220>
<221>  unsure
<222>  (704)

<220>
<221>  unsure
<222>  (731)

<220>
<221>  unsure
<222>  (748)

<220>
<221>  unsure
<222>  (768)

<220>
<221>  unsure
<222>  (772)

<220>
<221>  unsure
<222>  (781)

<220>
<221>  unsure
<222>  (785)

<220>
<221>  unsure
<222>  (803)

<220>
<221>  unsure
<222>  (806)..(807)

<220>
<221>  unsure
<222>  (819)

<220>
<221>  unsure
<222>  (825)

<220>
<221>  unsure
<222>  (830)

<220>
<221>  unsure
<222>  (839)

<400>  5
cggaacaggg agagtggtgt tcatgagcca ttctttcagg gaagaaacac aagagataaa  60
tctgaaactg ctgactcaaa cactgggtac tatgttggct taaacgttat agacattgac 120
aaagataatg gcaatccaca aatgtgtgct tcctatgctg cagagatata cagaaaccta 180
atggctgcag agcttataag gagacctaaa tcaaattaca tggagacttt gcaaagggat 240
atcacaaagg gcatgcgagg aatcctgatt gattgggctt tgaggttcct ggaggaatat 300
aaacttttgc cagacacact atacctcact gtatatctta ttgatcaatt tctttctcgg 360
aaatatattg aaagacagaa actacaactt cttggaataa ctagcatgct gattgcctca 420
aaatatgaag agatctgtgc gcctcgtgtt gaagaatttt gtttcataac tgataacaca 480
tatacaaaaa atcaggtgct gaaaatggag tgtgaagtgc ttaatgatct ggggtttcat 540
ctttcagttc ccacaatcaa aacgtttctg aggagattcc ttanagcagc acatgcttct 600
caaaaaagcc cttgggcaac tttgggctat ctggggcaat tatcttgccg gagttgacat 660
tgaccgatta cagttccctg aaattnaacc tcaatgggtg gaanctcggc gggtccctgc 720
aaaatggcac ncgacatcag actgcaangg aatccacctc gagcatanac tnaatcaaaa 780
nttangtatc aagatgcgta cgnatnnatg gaactgacna ggaanacaan ggatccccna 840
aggtata                                                           847

<210>  6
<211>  211
<212>  PRT
<213>  Triticum aestivum

<220>
<221>  UNSURE
<222>  (195)

<400>  6
Arg Asn Arg Glu Ser Gly Val His Glu Pro Phe Phe Gln Gly Arg Asn
1               5                   10                  15

Thr Arg Asp Lys Ser Glu Thr Ala Asp Ser Asn Thr Gly Tyr Tyr Val
            20                  25                  30

Gly Leu Asn Val Ile Asp Ile Asp Lys Asp Asn Gly Asn Pro Gln Met
        35                  40                  45

Cys Ala Ser Tyr Ala Ala Glu Ile Tyr Arg Asn Leu Met Ala Ala Glu
    50                  55                  60

Leu Ile Arg Arg Pro Lys Ser Asn Tyr Met Glu Thr Leu Gln Arg Asp
65                  70                  75                  80

Ile Thr Lys Gly Met Arg Gly Ile Leu Ile Asp Trp Ala Leu Arg Phe
                85                  90                  95

Leu Glu Glu Tyr Lys Leu Leu Pro Asp Thr Leu Tyr Leu Thr Val Tyr
            100                 105                 110

Leu Ile Asp Gln Phe Leu Ser Arg Lys Tyr Ile Glu Arg Gln Lys Leu
        115                 120                 125

Gln Leu Leu Gly Ile Thr Ser Met Leu Ile Ala Ser Lys Tyr Glu Glu
    130                 135                 140

Ile Cys Ala Pro Arg Val Glu Glu Phe Cys Phe Ile Thr Asp Asn Thr
145                 150                 155                 160

Tyr Thr Lys Asn Gln Val Leu Lys Met Glu Cys Glu Val Leu Asn Asp
                165                 170                 175

Leu Gly Phe His Leu Ser Val Pro Thr Ile Lys Thr Phe Leu Arg Arg
            180                 185                 190

Phe Leu Xaa Ala Ala His Ala Ser Gln Lys Ser Pro Trp Ala Thr Leu
        195                 200                 205

Gly Tyr Leu
    210

<210>  7
<211>  1007
<212>  DNA
<213>  Zea mays

<220>
<221>  unsure
<222>  (924)

<220>
<221>  unsure
<222>  (958)

<220>
<221>  unsure
<222>  (971)..(972)

<220>
<221>  unsure
<222>  (996)

<400>  7
gggagggaat tccttcctcc ttttctgttc ggcgccgtgc tcgcgcgcac ccacccgcac    60
gccccagtac ccccacgctg cacagtgcac gccgactttc ctccgccttg ctgctgcaag   120
tccgcaacca ctggaggaaa aatcttttcc ttcacttttc ttccctttcc ccccgcgcat   180
gcacgggctc tgattgacgc catgggggac gccgcggcct ccacgtccgc tcccaccacg   240
cccacctcca tcctcatctg cctggaagac ggcagcgacc ttctcgccga tgccgacgat   300
ggcgccggca ctgacctcgt tgtcgcccgc gacgaacgtc tgcttgtcgt ggaccaggac   360
gaggagtatg tagcgctgct cctgtccaag gagagcgcgt caggcggcgg cggcccggtg   420
gaggaaatgg aggactggat gaaggccgcg cgctccggat gcgtccgctg gatcatcaag   480
accacggcga tgttccggtt cggcgggaag accgcttacg tggccgtgaa ttacctcgat   540
cgcttcctgg cgcaacggcg agtcaatagg gagcatgcgt ggggtctgca gctgctcatg   600
gtggcgtgca tgtcgctggc gaccaagctg gaggagcacc acgctccgcg gctgtcggag   660
ttcccgctgg acgcgtgcga gttcgcgttc gacagcgcgt ccatcctgcg gatggagctc   720
ctcgtcctgg gcaccctcga gtggcggatg atcgccgtca cccccttccc ctacatcagc   780
tacttcgcgg cgcggttccg ggagacgagc gccgggcgaa tcctcatgcg cgccgtggag   840
tgcgtcttcg cggcgatcaa agtgataagc tcggtggagt aacggccgtc gaccatcgcc   900
gtggcatcca tcctcgtcgc gcgnggccgg gaggagactc ccgccggcag cctgggangc   960
gctcaaggcg nntcctcggg tcatcgtgcc cgcaantaga aaacggg                1007

<210>  8
<211>  238
<212>  PRT
<213>  Zea mays

<220>
<221>  UNSURE
<222>  (227)

<400> 8
Met Gly Asp Ala Ala Ala Ser Thr Ser Ala Pro Thr Thr Pro Thr Ser
1               5                   10                  15

Ile Leu Ile Cys Leu Glu Asp Gly Ser Asp Leu Leu Ala Asp Ala Asp
            20                  25                  30

Asp Gly Ala Gly Thr Asp Leu Val Val Ala Arg Asp Glu Arg Leu Leu
        35                  40                  45

Val Val Asp Gln Asp Glu Glu Tyr Val Ala Leu Leu Leu Ser Lys Glu
    50                  55                  60

Ser Ala Ser Gly Gly Gly Gly Pro Val Glu Glu Met Glu Asp Trp Met
65                  70                  75                  80

Lys Ala Ala Arg Ser Gly Cys Val Arg Trp Ile Ile Lys Thr Thr Ala
                85                  90                  95

Met Phe Arg Phe Gly Gly Lys Thr Ala Tyr Val Ala Val Asn Tyr Leu
            100                 105                 110

Asp Arg Phe Leu Ala Gln Arg Arg Val Asn Arg Glu His Ala Trp Gly
        115                 120                 125

Leu Gln Leu Leu Met Val Ala Cys Met Ser Leu Ala Thr Lys Leu Glu
    130                 135                 140

Glu His His Ala Pro Arg Leu Ser Glu Phe Pro Leu Asp Ala Cys Glu
145                 150                 155                 160

Phe Ala Phe Asp Ser Ala Ser Ile Leu Arg Met Glu Leu Leu Val Leu
                165                 170                 175

Gly Thr Leu Glu Trp Arg Met Ile Ala Val Thr Pro Phe Pro Tyr Ile
            180                 185                 190

Ser Tyr Phe Ala Ala Arg Phe Arg Glu Thr Ser Ala Gly Arg Ile Leu
        195                 200                 205

Met Arg Ala Val Glu Cys Val Phe Ala Ala Ile Lys Val Ile Ser Ser
    210                 215                 220

Val Glu Xaa Arg Pro Ser Thr Ile Ala Val Ala Ser Ile Leu
225                 230                 235

<210> 9
<211> 510
<212> DNA
<213> Oryza sativa

<220>
<221> unsure
<222> (424)

<220>
<221> unsure
<222> (441)

<400> 9
cttacagctt cctcctcgct ttgctggttg cagacgaacg cgggattccg gttcagcttg 60
aagacggcgt atgtcgcggt gacgtatctc gatcggttct tggcgcgccg gtgtgtcgat 120
agggacaagg agtgggcgct gcagctcctc tcggtggcgt gcctgtcgct ggcggcgaag 180
gtggaggagc gccggccgcc gcggctgccg gagttcaagc tggacatgta cgactgcgcg 240
tccttgatgc ggatggagct cctcgtcctc accacgctca agtggcagat gatcaccgag 300
acacccttct cctacctgaa ctgttcaccg cgaaattccg gcacgacgag cggaaggcat 360

cgtcctgcgc gccatcgaat gcatcttcgc tcgatcaaag tcatagctcg gtgggtacag 420
catnacgatc gctctagcag natctatcgt cggaacaagg agacggcgct aattagacga 480
ctaagtcgtc gtggctctat ggagcactaa 510

<210> 10
<211> 181
<212> PRT
<213> Oryza sativa

<400> 10
His Glu Leu Thr Ala Ser Ser Ser Leu Cys Trp Leu Gln Thr Asn Ala
1               5                   10                  15

Gly Phe Arg Phe Ser Leu Lys Thr Ala Tyr Val Ala Val Thr Tyr Leu
            20                  25                  30

Asp Arg Phe Leu Ala Arg Arg Cys Val Asp Arg Asp Lys Glu Trp Ala
        35                  40                  45

Leu Gln Leu Leu Ser Val Ala Cys Leu Ser Leu Ala Ala Lys Val Glu
    50                  55                  60

Glu Arg Arg Pro Pro Arg Leu Pro Glu Phe Lys Leu Asp Met Tyr Asp
65                  70                  75                  80

Cys Ala Ser Leu Met Arg Met Glu Leu Leu Val Leu Thr Thr Leu Lys
                85                  90                  95

Trp Gln Met Ile Thr Glu Thr Pro Phe Ser Tyr Leu Asn Cys Phe Thr
            100                 105                 110

Ala Lys Phe Arg His Asp Glu Arg Lys Ala Ile Val Leu Arg Ala Ile
        115                 120                 125

Glu Cys Ile Phe Ala Ser Ile Lys Val Ile Ser Ser Val Gly Tyr Gln
    130                 135                 140

Pro Ser Thr Ile Ala Leu Ala Ala Ile Leu Ile Ala Arg Asn Lys Glu
145                 150                 155                 160

Thr Ala Pro Asn Leu Asp Glu Leu Ser Val His Arg Leu Ala Pro Trp
                165                 170                 175

Gln Leu Met Met Leu
            180

<210> 11
<211> 2259
<212> DNA
<213> Glycine max

<400> 11
acaacttctc ccactcattc atcaacaacc acacacactc tctctcccct ctctgcacca 60
aaaccacttc tccggcgaca tctccggtca ggttccggca acctcatcgg cgaatcggca 120
tgccaacaca atgaatgcgg aacctccgct gccgccggcg ctcctcatgt cggtttcctg 180
cctctccgac tacgacctcc tctgcggcga ggactcctcc ggaatcctct ccggagagtc 240
gccggagtgc tccttctccg acatcgactc ctcacctcct ccgccgtcgc cgacgacaga 300
ggattgttat tcgatcgcga gcttcatcga gcacgagcgc aacttcgttc cgggattcga 360
gtacctgtcg cggttccaat ctcgctccct ggacgccaac gccagagaag aatcagttgg 420
atggattctc aaggtacacg cgtactatgg ctttcagcct ttgacggcgt acctcgccgt 480
caactatatg gatcggtttt tggattctcg ccggttgccg gaaacaaatg ggtggcctct 540
gcaacttgta tctgttgcat gcttgtcttt ggcagcaaag atggaagaac ctcttgttcc 600
atctctcttg gaccttcaga tagaaggtgc caagtacata tttgagccga gaacaattcg 660
taggatggag ctacttgttc tcggtgtctt agattggagg ctaagatcag taacaccact 720
ttgcttcctc gctttctttg cgtgcaaagt agattcaact ggaactttta tccggttcct 780
tatttccagg gcaacagaaa tcatcgtatc taatatccaa gaggctagct ttcttgctta 840

ctggccttca tgcattgctg ctgcagccat actcactgca gctaatgaaa ttcctaattg 900
gtctgtggtt aagcccgaaa atgctgagtc atggtgcgag ggactaagaa aagaaaaagt 960
aatagggtgc taccagttga tgcaagagct tgtgattaac aataaccaac ggaaactccc 1020
cttactaaaa gtgttgccgc agctgcgagt aacaactcgg acccgaatga ggtcaagtac 1080
tgtatcatca ttctcatcat cctcttcaac ctccttctcc ttgtcttgta agaggaggaa 1140
attaaataac cgtttgtggg tagatgacaa aggaaactcc gagtgaagag aaaacgaaca 1200
acaataataa aagaagggaa gaaaaagaga gggaataagg tgggccaagt tgtctagaaa 1260
cctcaacatt ttttagaggg tttttgcaat taaaaaatga cttgagtgag ggtgtagatt 1320
ataatagtat atatatgata tatctctatc gtatatacta agagagtttg atgggtttgg 1380
agtaattttt atttttatgt tggtgactta ttaatattga gtttgcagaa ttcacctagg 1440
gaagagggat tttgcgacat gttaccgtgg gagaggaaat gagagaagaa agaagtgaaa 1500
cactgaacca ggggtagaag aatttaatgt gatttgttct tgtaacctgt gattctgaag 1560
gaaagaattg agttgcgggc tggatttcaa agtttgcatt aattacttgg tgaaggagat 1620
gaaagatggt gggggcaacg ctgtagagat tgagaagaag aaaaagtaga gagaaggtat 1680
gaaaaactct ggtgattatt gaaagttgaa acttagaagt ttgaagtgtt caatgttcat 1740
atcatggtat tcataagtca agcaaagctt catttcttgg ccagcatcac tgcttcttca 1800
tcatccacgt tactactact ttgatgggac cctcaacagt aaagaacaat tgaagggcaa 1860
taagttgaag tttggctaca aatcgtggac tttttttgtt gggtattggc acgtgtgcag 1920
tcggttctgg tgcgtgccaa tgaagtgtgt acgtgtgatt tttctttttc ttggtttttc 1980
tttgcgggag ctgtttatat attttttcct atttttggcc atgagttttg gcctaactat 2040
acaggactcc aatggctggt gtccgcgcgt gtgatggaaa cacgtgtata tataggtttt 2100
aatttaaaaa ccttgaattt ttttatttgt tttcaagaga ggagaaccct ctttcacata 2160
ggggtaaaag gtctttgggg cctttttcct gtgtgctgtg tattggattg attaatatat 2220
aatgacaact attaatttct taaaaaaaaa aaaaaaaaa 2259

<210> 12
<211> 339
<212> PRT
<213> Glycine max

<400> 12
Met Ser Val Ser Cys Leu Ser Asp Tyr Asp Leu Leu Cys Gly Glu Asp
1               5                   10                  15

Ser Ser Gly Ile Leu Ser Gly Glu Ser Pro Glu Cys Ser Phe Ser Asp
            20                  25                  30

Ile Asp Ser Ser Pro Pro Pro Pro Ser Pro Thr Thr Glu Asp Cys Tyr
        35                  40                  45

Ser Ile Ala Ser Phe Ile Glu His Glu Arg Asn Phe Val Pro Gly Phe
    50                  55                  60

Glu Tyr Leu Ser Arg Phe Gln Ser Arg Ser Leu Asp Ala Asn Ala Arg
65                  70                  75                  80

Glu Glu Ser Val Gly Trp Ile Leu Lys Val His Ala Tyr Tyr Gly Phe
                85                  90                  95

Gln Pro Leu Thr Ala Tyr Leu Ala Val Asn Tyr Met Asp Arg Phe Leu
            100                 105                 110

Asp Ser Arg Arg Leu Pro Glu Thr Asn Gly Trp Pro Leu Gln Leu Val
        115                 120                 125

Ser Val Ala Cys Leu Ser Leu Ala Ala Lys Met Glu Glu Pro Leu Val
    130                 135                 140

Pro Ser Leu Leu Asp Leu Gln Ile Glu Gly Ala Lys Tyr Ile Phe Glu
145                 150                 155                 160

Pro Arg Thr Ile Arg Arg Met Glu Leu Leu Val Leu Gly Val Leu Asp
                165                 170                 175

Trp Arg Leu Arg Ser Val Thr Pro Leu Cys Phe Leu Ala Phe Phe Ala
            180                 185                 190

Cys Lys Val Asp Ser Thr Gly Thr Phe Ile Arg Phe Leu Ile Ser Arg
        195                 200                 205

Ala Thr Glu Ile Ile Val Ser Asn Ile Gln Glu Ala Ser Phe Leu Ala
    210                 215                 220

Tyr Trp Pro Ser Cys Ile Ala Ala Ala Ala Ile Leu Thr Ala Ala Asn
225                 230                 235                 240

Glu Ile Pro Asn Trp Ser Val Val Lys Pro Glu Asn Ala Glu Ser Trp
                245                 250                 255

Cys Glu Gly Leu Arg Lys Glu Lys Val Ile Gly Cys Tyr Gln Leu Met
            260                 265                 270

Gln Glu Leu Val Ile Asn Asn Asn Gln Arg Lys Leu Pro Leu Leu Lys
        275                 280                 285

Val Leu Pro Gln Leu Arg Val Thr Thr Arg Thr Arg Met Arg Ser Ser
    290                 295                 300

Thr Val Ser Ser Phe Ser Ser Ser Ser Ser Thr Ser Phe Ser Leu Ser
305                 310                 315                 320

Cys Lys Arg Arg Lys Leu Asn Asn Arg Leu Trp Val Asp Asp Lys Gly
                325                 330                 335

Asn Ser Glu

<210> 13
<211> 1994
<212> DNA
<213> Glycine max

<400> 13
gcacgagccg gaatcatgga ttcctcgccg gagtgttcct ccgacctcga ttcctcgccg 60
ccgtcggagg cggagtccat cgccggattc atggaagacg agcgcaactt cgtccccgga 120
ttcgaatacc tcaataggtt ccaatctcgc tctctcgacg cctctgccag agaagaatcc 180
gttgcatgga ttctcaaggt gcaggcttat tacgcttttc aaccggtcac ggcttatctt 240
tccgttaact acttggatag gttcttgaat tctcgaccgt tgccgccgaa aacgaatggg 300
tggccactgc aacttctctc tgttgcgtgc ttgtctttag cagcaaagat ggaggaatct 360
ctagttccat ctcttttgga ccttcaggta gaaggtgcta aatacgtatt tgaacccaaa 420
acaattagaa gaatggagct acttgttctg ggcgtgttgg attggaggct aagatcggtt 480
accccattta gcttcctcga tttctttgcg tgcaagttag attcaactgg gacttttacc 540
gggttcctca tttcacgtgc tacacaaatt atcttatcta atatacaaga ggctagcttt 600
cttgcgtatt ggccatcatg cattgctgca gcagccattc tccatgcagc aaatgaaatt 660
cctaattggt ctctcgttag gcctgagcat gcagagtcat ggtgtgaggg gttaagaaag 720
gagaaaatta tagggtgcta ccaattaatg caagaacttg tgattgacaa taaccagagg 780
aaacccccta aggtgttacc acagctgcga gtgacaatat ctcggcccat tatgaggtct 840
agtgtctcat ccttcttagc atcatcctct tcaccttcat catcctcttt gtcttgtaga 900
aggaggaaat taaataactc tttgtgggta gacgatgaca aaggaaactc ccaatgaaga 960
gaaaaagaac aataatagag gaggaaaaaa agaagaataa tgaaataagg tggtggacgg 1020
tggtccaagt tgtccagaaa ccgcaaaatt tttaagaggg tttttttgag tataaaatgg 1080
actagagagt cgaggtgtac attataatat agactatatt ttatgagagt tgcgagcttt 1140
tattattttg ttggtgggtg ttgccattca tcaatggcat tgcagattcc ctagggaagg 1200
ggattttgca aagtgtgtgt gggtgtgcgt gagagagagg tgggaaatta gatacaaaga 1260
tgcatttaat ggtttggtcg tgcaagcgtg gaagaaagaa gtgtgtagtt tggaattcaa 1320
agatgcgctt gttattggtg aaagagaaga gaatggtggt gggacattgc ttcagagctt 1380
ggaagaagaa aaaaagcata gtctcagagc agatatcaat agggtattga aagactttga 1440
agtttgagct gtttcttctt attataatgg ggtctctaag tcaagctact catcttggcc 1500
agcatgcctc gcttcttcag tttacacgtt acttttggta gtgtgatggg ataccactag 1560
agtacaaaaa aagaaatagc aataaggtga atcttatcac attttgtgga cttatgatga 1620
tacgtgtgag acgcctctgg tgtgtgtctc gcacgtgcct acgtgtgatt ttttattttt 1680
atttatgttt tagctatggc gggaaatgct ttttatttct ttatttcttt tttggcttga 1740

gctttggcct aactatacag gatccattgc ctggtgtcca cgcgtgatgg aaacacgtgt 1800
ctatagtttt cattttttgt tttggatttt ttcatttgtt ttcaagagag gagaaccctc 1860
ttttgttttc tttttagtgc ctaattggct ttgggagaaa ttggagtaaa ggcctttggg 1920
gccttttcct gagtgctttg tattgaattc attaataatg acactgttaa ttctataaaa 1980
aaaaaaaaaa aaaa 1994

<210> 14
<211> 318
<212> PRT
<213> Glycine max

<400> 14
Ala Arg Ala Gly Ile Met Asp Ser Ser Pro Glu Cys Ser Ser Asp Leu
1               5                   10                  15

Asp Ser Ser Pro Pro Ser Glu Ala Glu Ser Ile Ala Gly Phe Met Glu
            20                  25                  30

Asp Glu Arg Asn Phe Val Pro Gly Phe Glu Tyr Leu Asn Arg Phe Gln
        35                  40                  45

Ser Arg Ser Leu Asp Ala Ser Ala Arg Glu Glu Ser Val Ala Trp Ile
    50                  55                  60

Leu Lys Val Gln Ala Tyr Tyr Ala Phe Gln Pro Val Thr Ala Tyr Leu
65                  70                  75                  80

Ser Val Asn Tyr Leu Asp Arg Phe Leu Asn Ser Arg Pro Leu Pro Pro
                85                  90                  95

Lys Thr Asn Gly Trp Pro Leu Gln Leu Leu Ser Val Ala Cys Leu Ser
            100                 105                 110

Leu Ala Ala Lys Met Glu Glu Ser Leu Val Pro Ser Leu Leu Asp Leu
        115                 120                 125

Gln Val Glu Gly Ala Lys Tyr Val Phe Glu Pro Lys Thr Ile Arg Arg
    130                 135                 140

Met Glu Leu Leu Val Leu Gly Val Leu Asp Trp Arg Leu Arg Ser Val
145                 150                 155                 160

Thr Pro Phe Ser Phe Leu Asp Phe Phe Ala Cys Lys Leu Asp Ser Thr
                165                 170                 175

Gly Thr Phe Thr Gly Phe Leu Ile Ser Arg Ala Thr Gln Ile Ile Leu
            180                 185                 190

Ser Asn Ile Gln Glu Ala Ser Phe Leu Ala Tyr Trp Pro Ser Cys Ile
        195                 200                 205

Ala Ala Ala Ala Ile Leu His Ala Ala Asn Glu Ile Pro Asn Trp Ser
    210                 215                 220

Leu Val Arg Pro Glu His Ala Glu Ser Trp Cys Glu Gly Leu Arg Lys
225                 230                 235                 240

Glu Lys Ile Ile Gly Cys Tyr Gln Leu Met Gln Glu Leu Val Ile Asp
                245                 250                 255

Asn Asn Gln Arg Lys Pro Pro Lys Val Leu Pro Gln Leu Arg Val Thr
            260                 265                 270

Ile Ser Arg Pro Ile Met Arg Ser Ser Val Ser Ser Phe Leu Ala Ser
        275                 280                 285

Ser Ser Ser Pro Ser Ser Ser Ser Leu Ser Cys Arg Arg Arg Lys Leu
    290                 295                 300

Asn Asn Ser Leu Trp Val Asp Asp Asp Lys Gly Asn Ser Gln
305                 310                 315

<210>  15
<211>  570
<212>  DNA
<213>  Triticum aestivum

<220>
<221>  unsure
<222>  (499)

<220>
<221>  unsure
<222>  (515)..(516)

<220>
<221>  unsure
<222>  (558)

<400>  15
acagaggttc acctaaaaaa aggctagcag ttcttcccaa agagacaaca gttctaagaa  60
aagaagcagg agctgcagct ggtgagcgtc tgtgccctgc tgattgcgtg caagtacgaa 120
gagatttggg ctccagaggt gaacgacttc atattgttct ccgacaacac atatactagg 180
gagcagattc tgaggatgga gaaggcaatc ctgaacatgc ttgagtggaa cctgacagtg 240
cccacacctt acgtcttcct cgtgtgattc gccaaggccg catcctcctg agataagaag 300
aacggcaagg aggtaaaagg aacaccagat tttaacaaat cctcagatgt agtacgtatc 360
tccatttgcc aaacatgatc tattgctgaa ttctgttctc cctggtgtat tgtctaaatg 420
gagacacgtc tttttttcgt ggactggcgc tctgtagtat ggacagaata tgtttgattc 480
agcacacaag agacaggtna tcaacacaca gtagnnacag tgtctgtaca gccgtataca 540
taacattata cttctcanag accactttgg                                  570

<210>  16
<211>  75
<212>  PRT
<213>  Triticum aestivum

<220>
<221>  UNSURE
<222>  (68)

<400>  16
Lys Gln Glu Leu Gln Leu Val Ser Val Cys Ala Leu Leu Ile Ala Cys
1               5                   10                  15

Lys Tyr Glu Glu Ile Trp Ala Pro Glu Val Asn Asp Phe Ile Leu Phe
            20                  25                  30

Ser Asp Asn Thr Tyr Thr Arg Glu Gln Ile Leu Arg Met Glu Lys Ala
        35                  40                  45

Ile Leu Asn Met Leu Glu Trp Asn Leu Thr Val Pro Thr Pro Tyr Val
    50                  55                  60

Phe Leu Val Xaa Phe Ala Lys Ala Ala Ser Ser
65                  70                  75

<210>  17
<211>  1932
<212>  DNA
<213>  Zea mays

<220>
<221> unsure
<222> (8)

<220>
<221> unsure
<222> (26)

<220>
<221> unsure
<222> (159)

<400> 17
gccacaantg caccgcagac sgcacntsgg cctccctcct ccgtccgtcc gtcctcttcc 60
ttgtgccttg tcctcctcca ctcgccactg ccgcattctg cccaagtccc aaacacgcgc 120
accagccacc cagcactcca gccgccagac cagagtctnc ggcgccgccg tcgcacgaca 180
ggagagggag agatacgcgg gctttgactt gccgccggtg cgtccgtgcg tgcctggtgg 240
gaatagtggg agacgccggt acagtacagg agccatggcg ccgagctgct acgacgcggc 300
agcgtccatg ctcctctgcg ccgaggagca cagcagcatc ctgtggtacg aggaggagga 360
ggaggagctg gaggcggtcg ggagaaggag cggccggtcg ccgggctacg gggacgactt 420
cggcgcggac ttgttcccgc cgcagtcgga ggaatgcgtg gccggtctgg tggagcggga 480
acgggaccac atgccggggc cgtgctacgg cgacaggctg cgcggcggcg gcggctgtct 540
ctgcgtccgc cgggaggccg tcgactggat ttggaaggct tacacgcacc acaggttccg 600
ccctctcact gcctacttgg cagtgaacta cctcgatcgc ttcctctcgc tgtctgaggt 660
gccggactgc aaggactgga tgacgcagct cctcgcggtg gcgtgcgttt ctctggccgc 720
caagatggag gaaaccgccg tcccgcagtg cctggacctt caggaggtcg gagacgcgcg 780
gtacgtgttc gaggcgaaga cggtccagag gatggagctc ctggttctaa caaccctcaa 840
ctggaggatg catgccgtga cgccgttctc ctacgtggat tacttcctga acaagctcaa 900
caacggcggc agcacggcgc cgaggagctg ctggctcttg cagtccgcgg agcttatctt 960
gcgtgcggcc agaggaaccg gctgcgtcgg gttcaggccg tccgagatcg ccgccgcggt 1020
tgcagccgcc gtggccggag acgtggacga cgcggacggc gtcgagaacg cctgctgcgc 1080
tcacgtagat aaggagcggg tgttgcggtg ccaggaagcg atcgggtcca tggcgtcctc 1140
ggcggccatt gacgacgcta ccgtgccacc gaaatctgcg agacgcagga gctcccccgt 1200
gcccgtgccg cagagccctg tgggggtgct ggacgcggct ccctgcctca gctacaggag 1260
cgaagaggca gcgactgcga ctgcgactgc gacttctgct gcctcacatg gggcccctgg 1320
ctcttcaagc tcgtcctcga cctccccggt gaccagcaaa aggaggaaac tcgccagccg 1380
atgtgatgga tcgtgcagtg accggtcaaa gcgcgcgccc gcccaatgga ccaaagagtg 1440
aattgactag ggcgtctgct tgctttctga tcaaagagtg cattgagagg cggcaaaatg 1500
gaggaataaa ggggattttg gcatgacgag ggcaaaggag ttgatgaata aagacgcgac 1560
gaggtggaca acacctaaat tgccgatctt ttctttgcaa ggggagtagg ggacctgctt 1620
gggcctgggg gagggtagta gacagcccag cacaaaaacc tggttggtgt gccacgacca 1680
caatcggcgc gccagccatg gctttgtagg aaacacaagg gcgctagagg agatccgatg 1740
ggatgactca gaataaagat agtggaggga ccagaccgta tgcagtatgt gcaacaacta 1800
ggcactggca tgcttatgct caagtaatct gataacttga atgttgtgta tccgacaaac 1860
tgcttctgtg aagagagaaa tgcaggtaga cgatgaatgt atgtgaaaaa aaaaaaaaaa 1920
aaaaaaaaaa ac 1932

<210> 18
<211> 388
<212> PRT
<213> Zea mays

<400> 18
Met Ala Pro Ser Cys Tyr Asp Ala Ala Ala Ser Met Leu Leu Cys Ala
1               5                   10                  15

Glu Glu His Ser Ser Ile Leu Trp Tyr Glu Glu Glu Glu Glu Glu Leu
            20                  25                  30

Glu Ala Val Gly Arg Arg Ser Gly Arg Ser Pro Gly Tyr Gly Asp Asp
        35                  40                  45

Phe Gly Ala Asp Leu Phe Pro Pro Gln Ser Glu Glu Cys Val Ala Gly
    50                  55                  60

Leu Val Glu Arg Glu Arg Asp His Met Pro Gly Pro Cys Tyr Gly Asp
65                  70                  75                  80

Arg Leu Arg Gly Gly Gly Gly Cys Leu Cys Val Arg Arg Glu Ala Val
                85                  90                  95

Asp Trp Ile Trp Lys Ala Tyr Thr His His Arg Phe Arg Pro Leu Thr
            100                 105                 110

Ala Tyr Leu Ala Val Asn Tyr Leu Asp Arg Phe Leu Ser Leu Ser Glu
        115                 120                 125

Val Pro Asp Cys Lys Asp Trp Met Thr Gln Leu Leu Ala Val Ala Cys
    130                 135                 140

Val Ser Leu Ala Ala Lys Met Glu Glu Thr Ala Val Pro Gln Cys Leu
145                 150                 155                 160

Asp Leu Gln Glu Val Gly Asp Ala Arg Tyr Val Phe Glu Ala Lys Thr
                165                 170                 175

Val Gln Arg Met Glu Leu Leu Val Leu Thr Thr Leu Asn Trp Arg Met
            180                 185                 190

His Ala Val Thr Pro Phe Ser Tyr Val Asp Tyr Phe Leu Asn Lys Leu
        195                 200                 205

Asn Asn Gly Gly Ser Thr Ala Pro Arg Ser Cys Trp Leu Leu Gln Ser
    210                 215                 220

Ala Glu Leu Ile Leu Arg Ala Ala Arg Gly Thr Gly Cys Val Gly Phe
225                 230                 235                 240

Arg Pro Ser Glu Ile Ala Ala Ala Val Ala Ala Ala Val Ala Gly Asp
                245                 250                 255

Val Asp Asp Ala Asp Gly Val Glu Asn Ala Cys Cys Ala His Val Asp
            260                 265                 270

Lys Glu Arg Val Leu Arg Cys Gln Glu Ala Ile Gly Ser Met Ala Ser
        275                 280                 285

Ser Ala Ala Ile Asp Asp Ala Thr Val Pro Pro Lys Ser Ala Arg Arg
    290                 295                 300

Arg Ser Ser Pro Val Pro Val Pro Gln Ser Pro Val Gly Val Leu Asp
305                 310                 315                 320

Ala Ala Pro Cys Leu Ser Tyr Arg Ser Glu Glu Ala Ala Thr Ala Thr
                325                 330                 335

Ala Thr Ala Thr Ser Ala Ala Ser His Gly Ala Pro Gly Ser Ser Ser
            340                 345                 350

Ser Ser Ser Thr Ser Pro Val Thr Ser Lys Arg Arg Lys Leu Ala Ser
        355                 360                 365

Arg Cys Asp Gly Ser Cys Ser Asp Arg Ser Lys Arg Ala Pro Ala Gln
    370                 375                 380

Trp Thr Lys Glu
385

<210>  19
<211>  481

<212>  DNA
<213>  Oryza sativa

<220>
<221>  unsure
<222>  (88)

<220>
<221>  unsure
<222>  (130)

<220>
<221>  unsure
<222>  (251)

<220>
<221>  unsure
<222>  (311)

<220>
<221>  unsure
<222>  (352)

<220>
<221>  unsure
<222>  (359)

<220>
<221>  unsure
<222>  (394)

<220>
<221>  unsure
<222>  (400)

<220>
<221>  unsure
<222>  (408)

<220>
<221>  unsure
<222>  (410)

<220>
<221>  unsure
<222>  (415)

<220>
<221>  unsure
<222>  (420)

<220>
<221>  unsure
<222>  (426)

<220>
<221>  unsure
<222>  (432)..(433)

<220>
<221>  unsure
<222>  (448)

<220>
<221>  unsure
<222>  (457)

<220>
<221> unsure
<222> (461)..(462)

<220>
<221> unsure
<222> (470)

<220>
<221> unsure
<222> (475)

<400> 19
cttacatgta agctcgtgcc gaattcggca cgagcttaca cgagcgcaac ccatgggcgc 60
cgcggctgct cgccatcagc tgcctcancc tcgccgccaa gatgcagcgc gccgccgcca 120
tctccgccgn cgacatccag aggggcgagg agttcatgtt cgacgaggcg aaaatccagc 180
gcatggagca gatggtgctc aacgcgctgg agtggcggac gcgctccgtc acgccgctcg 240
ccttcctcgg nttctttctc tccgcgtggt tcccgcaagc cgcggcaccc ggcgctgctc 300
gatgccatca nggccgcgcc gtcgagctcc tcctccgcgt ctaagccggg angtgaacna 360
tggtgggagt tctccccctt cggtggccgg ccgncgcggn tctcctcncn gccgncggan 420
aaggcntccg gnngcccaaa ctccttcnct tccaaanctg nnggccccgn tttgncccct 480
t 481

<210> 20
<211> 110
<212> PRT
<213> Oryza sativa

<220>
<221> UNSURE
<222> (26)

<220>
<221> UNSURE
<222> (40)

<220>
<221> UNSURE
<222> (100)

<400> 20
Ala Arg Ala Glu Phe Gly Thr Ser Leu His Glu Arg Asn Pro Trp Ala
1               5                   10                  15

Pro Arg Leu Leu Ala Ile Ser Cys Leu Xaa Leu Ala Ala Lys Met Gln
            20                  25                  30

Arg Ala Ala Ala Ile Ser Ala Xaa Asp Ile Gln Arg Gly Glu Glu Phe
        35                  40                  45

Met Phe Asp Glu Ala Lys Ile Gln Arg Met Glu Gln Met Val Leu Asn
    50                  55                  60

Ala Leu Glu Trp Arg Thr Arg Ser Val Thr Pro Leu Ala Phe Leu Gly
65                  70                  75                  80

Phe Phe Leu Ser Ala Trp Phe Pro Gln Ala Ala Ala Pro Gly Ala Ala
                85                  90                  95

Arg Cys His Xaa Gly Arg Ala Val Glu Leu Leu Leu Arg Val
            100                 105                 110

<210> 21
<211> 789

<212>  DNA
<213>  Triticum aestivum

<400>  21
cacctgaggg cgactcgagg gtgccctcgc cccgtccgcc gtgaccaccc ctcttcggat  60
ctcaccgcct cgaccaaaat gtgatttgag gcaaattctg cgtttgaggc aaggacaata 120
aaagtgatgg agcttttggt cttcagcacc ttgaaatgga ggatgcaagc tgttactgct 180
tgctcgttta ttgactactt cctttgcaaa ttcaatgatc atgacacacc ctccatgctt 240
gcattctcct gctcaactga cctcatcctg agcacaacta agtgagctga ttttttggtg 300
ttcagacatt cagagattgc tggaagtgtt gcacttcctt catttgggga gcacaagact 360
tcagttgtcg aaatggctac aactaattgc aagtatataa acaagggagt gtgatgtgac 420
aggaaagatc ctgatgaagt gcttccttta tggaatgcct atctgaagtt tggactaaga 480
gacatgcttt aattggctta gtaaaaaata cttgctaaag agaaataaga ttcaaagtag 540
atgtttttat tgtagattag gatatgtgtg ttctgccacc ggttcgactt ctcatattag 600
aaggcaagca gttagttcat atcttactac tttgcactat tgtagatgga tggtgaggga 660
ttgagaggct actactatta atgtgcgtaa actttgcatc tttagctctc taaatgaaac 720
cggtgatggt taacctgaaa aaaaaaaaaa aaaaaaaaaa aaaaaaaaaa aaaaaaaaaa 780
aaaaaaaaa

<210>  22
<211>  163
<212>  PRT
<213>  Triticum aestivum

<220>
<221>  UNSURE
<222>  (28)

<220>
<221>  UNSURE
<222>  (95)

<220>
<221>  UNSURE
<222>  (139)

<400>  22
His Leu Arg Ala Thr Arg Gly Cys Pro Arg Pro Val Arg Arg Asp His
1               5                   10                  15

Pro Ser Ser Asp Leu Thr Ala Ser Thr Lys Met Xaa Phe Glu Ala Asn
            20                  25                  30

Ser Ala Phe Glu Ala Arg Thr Ile Lys Val Met Glu Leu Leu Val Phe
        35                  40                  45

Ser Thr Leu Lys Trp Arg Met Gln Ala Val Thr Ala Cys Ser Phe Ile
    50                  55                  60

Asp Tyr Phe Leu Cys Lys Phe Asn Asp His Asp Thr Pro Ser Met Leu
65                  70                  75                  80

Ala Phe Ser Cys Ser Thr Asp Leu Ile Leu Ser Thr Thr Lys Xaa Ala
                85                  90                  95

Asp Phe Leu Val Phe Arg His Ser Glu Ile Ala Gly Ser Val Ala Leu
            100                 105                 110

Pro Ser Phe Gly Glu His Lys Thr Ser Val Val Glu Met Ala Thr Thr
        115                 120                 125

Asn Cys Lys Tyr Ile Asn Lys Gly Val Xaa Cys Asp Arg Lys Asp Pro
    130                 135                 140

Asp Glu Val Leu Pro Leu Trp Asn Ala Tyr Leu Lys Phe Gly Leu Arg
145                 150                 155                 160

Asp Met Leu

<210> 23
<211> 603
<212> DNA
<213> Zea mays

<220>
<221> unsure
<222> (441)

<220>
<221> unsure
<222> (447)

<220>
<221> unsure
<222> (485)

<220>
<221> unsure
<222> (498)

<220>
<221> unsure
<222> (528)

<220>
<221> unsure
<222> (553)

<220>
<221> unsure
<222> (560)

<220>
<221> unsure
<222> (576)..(577)

<220>
<221> unsure
<222> (598)

<400> 23
aacagaattc ggcacgagcc gcggtcggct gggtttcacg cgccgcggcg cggctaggct 60
tctccgcgct caccgccgcg ctcgccgccg cctacctcga ccgctgcttc ctccccgggg 120
gcgcgctccg gctcggcgac cagccctgga tggcgcgcct agccgccgtc acctgcttcg 180
cgctcgccgc caaggtcgag gagacgcgcg tgccgccgct cctcgacctc cagctctacg 240
ccgccgctga cgccgcggat ccgtacgtat tcgaggccaa gacggtgcgc cggatggagc 300
tgctcgtgct ctccgcgctt gggtggcgga tgcaccctgt cacgcccttc tcctacctcc 360
agcccgtcct cgccgacgct gcgacgcgcc tgcgtagctg cgagggcgtc ctgctcgcgg 420
tcatggccga ctggaggtgg cctcggcacc ggccttcggc gtgggccgcc gccgcgttgc 480
tgatcacagc cgccgccggc gacggcggcg acggcgacgg cgacacggag ctcctggcgc 540
tcatcaatgc ccccgaggac aagaccgccg agtgtgccaa gatcatctcc gaggtgacgg 600
gcatgagctt cctcgcctgc gatgtcggcg tgagcgccgg aaataagcgt aagcacgcgg 660
cggcgcagtt gtactcgccg ccgccgagcc cgagcggcgt gatcggcgcg ctgtcctgct 720
tcagctgcga gagctcgacg tccgccaccg ctatggctgc ggcggtcggc ccgtgggcgc 780
cgtcggcgtc cgtgtccgtg tcgtcctctc cagagccacc aggtcgggcc cccaagcgcg 840
cagcggcggc gtcggcgtcg gcgtcggcgt cagccggggt cgcgccaccg gtccaggtcc 900
cgcatcagct accccccgac gaggagagcc gcgacgcctg gccgtccacc tgcgccgcgt 960
gacgcaccgt gccggaaacg gtgcctatgg cgagaccgcc gttcggtggc ggtggagaat 1020
ggagaacaag gagcatcatt ggctcgcgtc ggtgagcagg agaacgaact atttgcccca 1080
ttgccgtgac agatgggggg tgttcactgc gtggagccgc gctgancaat ga 1132

<210> 24
<211> 318
<212> PRT
<213> Zea mays

<400> 24
Asn Ser Ala Arg Ala Ala Val Gly Trp Val Ser Arg Ala Ala Ala Arg
1               5                   10                  15

Leu Gly Phe Ser Ala Leu Thr Ala Ala Leu Ala Ala Ala Tyr Leu Asp
            20                  25                  30

Arg Cys Phe Leu Pro Gly Gly Ala Leu Arg Leu Gly Asp Gln Pro Trp
        35                  40                  45

Met Ala Arg Leu Ala Ala Val Thr Cys Phe Ala Leu Ala Ala Lys Val
    50                  55                  60

Glu Glu Thr Arg Val Pro Pro Leu Leu Asp Leu Gln Leu Tyr Ala Ala
65                  70                  75                  80

Ala Asp Ala Ala Asp Pro Tyr Val Phe Glu Ala Lys Thr Val Arg Arg
                85                  90                  95

Met Glu Leu Leu Val Leu Ser Ala Leu Gly Trp Arg Met His Pro Val
            100                 105                 110

Thr Pro Phe Ser Tyr Leu Gln Pro Val Leu Ala Asp Ala Ala Thr Arg
        115                 120                 125

Leu Arg Ser Cys Glu Gly Val Leu Leu Ala Val Met Ala Asp Trp Arg
    130                 135                 140

Trp Pro Arg His Arg Pro Ser Ala Trp Ala Ala Ala Ala Leu Leu Ile
145                 150                 155                 160

Thr Ala Ala Ala Gly Asp Gly Gly Asp Gly Asp Gly Asp Thr Glu Leu
                165                 170                 175

Leu Ala Leu Ile Asn Ala Pro Glu Asp Lys Thr Ala Glu Cys Ala Lys
            180                 185                 190

Ile Ile Ser Glu Val Thr Gly Met Ser Phe Leu Ala Cys Asp Val Gly
        195                 200                 205

Val Ser Ala Gly Asn Lys Arg Lys His Ala Ala Ala Gln Leu Tyr Ser
    210                 215                 220

Pro Pro Pro Ser Pro Ser Gly Val Ile Gly Ala Leu Ser Cys Phe Ser
225                 230                 235                 240

Cys Glu Ser Ser Thr Ser Ala Thr Ala Met Ala Ala Ala Val Gly Pro
                245                 250                 255

Trp Ala Pro Ser Ala Ser Val Ser Val Ser Ser Ser Pro Glu Pro Pro
            260                 265                 270

Gly Arg Ala Pro Lys Arg Ala Ala Ala Ala Ser Ala Ser Ala Ser Ala
        275                 280                 285

Ser Ala Gly Val Ala Pro Pro Val Gln Val Pro His Gln Leu Pro Pro
    290                 295                 300

Asp Glu Glu Ser Arg Asp Ala Trp Pro Ser Thr Cys Ala Ala
305                 310                 315

<210>  25
<211>  674
<212>  DNA
<213>  Glycine max

<220>
<221>  unsure
<222>  (527)

<220>
<221>  unsure
<222>  (561)

<220>
<221>  unsure
<222>  (640)

<220>
<221>  unsure
<222>  (643)

<400>  25
cactcactca ccccttcctt tctaactcct caaattgtgt gttctgagaa tggaaatgcc  60
tccttctcca tcggggcatt ccgcactctc catcccataa aagtcccaga tccaagatgg 120
cttaccacca tcaaaaatcc cttttggaca ccctatactg ctccgaagag cattggatag 180
gggaaggtga atttgaccaa gcagaggagg agtacggtaa cagtaatagc aatagtagca 240
gcaccttagt aaacaactcc cctgagtcct cccctcattt gttgctcgaa agcgacatgt 300
tttgggacga acaagagttg gcatcgctgt tggagaaaga acaacacaac ccactaagca 360
cttgctgtct ccaaagcaac cctgccttgg agggtgctcg catagaagcc gtggagtgga 420
ttctcaaagt aaacgcccac tactccttct ctgccctcac cgctgttctt gctgtcaact 480
actttgaccg ttttctcttc agcttccgct ttcagaatga cattaancca tggatgactc 540
ggggtcgctg ccgtcgcttg nctctccctc gctgccaaag tgggcgagac acacgttccc 600
tttcttattt gacccttcaa caaagtggga ggaggagtan atnctttgtt ccaagccaaa 660
gacgattaaa aaag                                                   674

<210>  26
<211>  186
<212>  PRT
<213>  Glycine max

<220>
<221>  UNSURE
<222>  (137)

<220>
<221>  UNSURE
<222>  (149)

<220>
<221>  UNSURE
<222>  (175)..(176)

<400>  26
Met Ala Tyr His His Gln Lys Ser Leu Leu Asp Thr Leu Tyr Cys Ser
1               5                   10                  15

Glu Glu His Trp Ile Gly Glu Gly Glu Phe Asp Gln Ala Glu Glu Glu
            20                  25                  30

Tyr Gly Asn Ser Asn Ser Asn Ser Ser Ser Thr Leu Val Asn Asn Ser
        35                  40                  45

Pro Glu Ser Ser Pro His Leu Leu Leu Glu Ser Asp Met Phe Trp Asp
    50                  55                  60

Glu Gln Glu Leu Ala Ser Leu Leu Glu Lys Glu Gln His Asn Pro Leu
65                  70                  75                  80

Ser Thr Cys Cys Leu Gln Ser Asn Pro Ala Leu Glu Gly Ala Arg Ile
                85                  90                  95

Glu Ala Val Glu Trp Ile Leu Lys Val Asn Ala His Tyr Ser Phe Ser
            100                 105                 110

Ala Leu Thr Ala Val Leu Ala Val Asn Tyr Phe Asp Arg Phe Leu Phe
        115                 120                 125

Ser Phe Arg Phe Gln Asn Asp Ile Xaa Pro Trp Met Thr Arg Gly Arg
    130                 135                 140

Cys Arg Arg Leu Xaa Leu Pro Arg Cys Gln Ser Gly Arg Asp Thr Arg
145                 150                 155                 160

Ser Leu Ser Tyr Leu Thr Leu Gln Gln Ser Gly Arg Arg Ser Xaa Xaa
                165                 170                 175

Phe Val Pro Ser Gln Arg Arg Leu Lys Lys
            180                 185

<210>  27
<211>  554
<212>  DNA
<213>  Glycine max

<400>  27
ctccctttca cctttcttca tagcctacca cttttctgct ttcatctact ctcacttctc  60
ttcacacact gagacacaca gagagagaaa aataaagggt gtgatgggtg tcttactgag 120
tgttttcttt ttataatgaa caaagaactg cacaccctct tcttcaccga agaagaagat 180
ggcaattcag caccacaatg accaactaga gcataatgaa aatgtctcat ctgtccttga 240
tgccctttac tgtgacgaag gaaagtggga agaggaagag gaggagaaag aagaagaaga 300
agatgaaggt gaaaatgaaa gtgaagtgac aacaaacact gcaacttgtc ttttccctct 360
gctcttgttg gagcaagact tgttctggga agatgaggaa ctaaactcta tcttttccaa 420
agagaaggtt caacatgaag aagcctatgg tataacaatc tgaacagtga tgtgtataac 480
aacaacaaca atactagtat ataatgtgat ttggctcttg ctcttcagct cgtcggagcg 540
tgatgatgct gaat                                                   554

<210>  28
<211>  94
<212>  PRT
<213>  Glycine max

<400>  28
Met Ala Ile Gln His His Asn Asp Gln Leu Glu His Asn Glu Asn Val
1               5                   10                  15

Ser Ser Val Leu Asp Ala Leu Tyr Cys Asp Glu Gly Lys Trp Glu Glu
            20                  25                  30

Glu Glu Glu Glu Lys Glu Glu Glu Glu Asp Glu Gly Glu Asn Glu Ser
        35                  40                  45

Glu Val Thr Thr Asn Thr Ala Thr Cys Leu Phe Pro Leu Leu Leu Leu
    50                  55                  60

Glu Gln Asp Leu Phe Trp Glu Asp Glu Glu Leu Asn Ser Ile Phe Ser
65                  70                  75                  80

Lys Glu Lys Val Gln His Glu Glu Ala Tyr Gly Ile Thr Ile
                85                  90

<210>  29
<211>  372
<212>  PRT
<213>  Catharanthus roseus

<400>  29

Met Ala Asp Lys Glu Asn Cys Ile Arg Val Thr Arg Leu Ala Lys Lys
1               5                   10                  15

Arg Ala Val Glu Ala Met Ala Ala Ser Glu Gln Gln Arg Pro Ser Lys
            20                  25                  30

Lys Arg Val Val Leu Gly Glu Leu Lys Asn Leu Ser Ser Asn Ile Ser
        35                  40                  45

Ser Ile Gln Thr Tyr Asp Phe Ser Ser Gly Pro Gln Lys Gln Gln Lys
    50                  55                  60

Asn Lys Asn Lys Arg Lys Ala Lys Glu Ser Leu Gly Phe Glu Val Lys
65                  70                  75                  80

Glu Lys Lys Val Glu Glu Ala Gly Ile Asp Val Phe Ser Gln Ser Asp
                85                  90                  95

Asp Pro Gln Met Cys Gly Ala Tyr Val Ser Asp Ile Tyr Glu Tyr Leu
            100                 105                 110

His Lys Met Glu Met Glu Thr Lys Arg Arg Pro Leu Pro Asp Tyr Leu
        115                 120                 125

Asp Lys Val Gln Lys Asp Val Thr Ala Asn Met Arg Gly Val Leu Ile
    130                 135                 140

Asp Trp Leu Val Glu Val Ala Glu Glu Tyr Lys Leu Leu Pro Asp Thr
145                 150                 155                 160

Leu Tyr Leu Thr Val Ser Tyr Ile Asp Arg Phe Leu Ser Met Asn Ala
                165                 170                 175

Leu Ser Arg Gln Lys Leu Gln Leu Leu Gly Val Ser Ser Met Leu Ile
            180                 185                 190

Ala Ser Lys Tyr Glu Glu Ile Ser Pro Pro His Val Glu Asp Phe Cys
        195                 200                 205

Tyr Ile Thr Asp Asn Thr Tyr Lys Lys Glu Glu Val Val Lys Met Glu
    210                 215                 220

Ala Asp Val Leu Lys Phe Leu Lys Phe Glu Met Gly Asn Pro Thr Ile
225                 230                 235                 240

Lys Thr Phe Leu Arg Arg Leu Thr Arg Val Val Gln Asp Gly Asp Lys
                245                 250                 255

Asn Pro Asn Leu Gln Phe Glu Phe Leu Gly Tyr Tyr Leu Ala Glu Leu
            260                 265                 270

Ser Leu Leu Asp Tyr Gly Cys Val Lys Phe Leu Pro Ser Leu Ile Ala
        275                 280                 285

Ser Ser Val Ile Phe Leu Ser Arg Phe Thr Leu Gln Pro Lys Val His
    290                 295                 300

Pro Trp Asn Ser Leu Leu Gln His Asn Ser Gly Tyr Lys Pro Ala Asp
305                 310                 315                 320

Leu Lys Glu Cys Val Leu Ile Ile His Asp Leu Gln Leu Ser Lys Arg
                325                 330                 335

Gly Ser Ser Leu Val Ala Val Arg Asp Lys Tyr Lys Gln His Lys Phe
            340                 345                 350

Lys Cys Val Ser Thr Leu Thr Ala Pro Pro Ser Ile Pro Asp Glu Phe
        355                 360                 365

Phe Glu Asp Ile
    370

<210>  30
<211>  335
<212>  PRT
<213>  Arabidopsis thaliana

<400>  30
Met Arg Ser Tyr Arg Phe Ser Asp Tyr Leu His Met Ser Val Ser Phe
  1               5                  10                  15

Ser Asn Asp Met Asp Leu Phe Cys Gly Glu Asp Ser Gly Val Phe Ser
            20                  25                  30

Gly Glu Ser Thr Val Asp Phe Ser Ser Ser Glu Val Asp Ser Trp Pro
        35                  40                  45

Gly Asp Ser Ile Ala Cys Phe Ile Glu Asp Glu Arg His Phe Val Pro
    50                  55                  60

Gly His Asp Tyr Leu Ser Arg Phe Gln Thr Arg Ser Leu Asp Ala Ser
 65                  70                  75                  80

Ala Arg Glu Asp Ser Val Ala Trp Ile Leu Lys Val Gln Ala Tyr Tyr
                85                  90                  95

Asn Phe Gln Pro Leu Thr Ala Tyr Leu Ala Val Asn Tyr Met Asp Arg
            100                 105                 110

Phe Leu Tyr Ala Arg Arg Leu Pro Glu Thr Ser Gly Trp Pro Met Gln
        115                 120                 125

Leu Leu Ala Val Ala Cys Leu Ser Leu Ala Ala Lys Met Glu Glu Ile
    130                 135                 140

Leu Val Pro Ser Leu Phe Asp Phe Gln Val Ala Gly Val Lys Tyr Leu
145                 150                 155                 160

Phe Glu Ala Lys Thr Ile Lys Arg Met Glu Leu Leu Val Leu Ser Val
                165                 170                 175

Leu Asp Trp Arg Leu Arg Ser Val Thr Pro Phe Asp Phe Ile Ser Phe
            180                 185                 190

Phe Ala Tyr Lys Ile Asp Pro Ser Gly Thr Phe Leu Gly Phe Phe Ile
        195                 200                 205

Ser His Ala Thr Glu Ile Ile Leu Ser Asn Ile Lys Glu Ala Ser Phe
    210                 215                 220

Leu Glu Tyr Trp Pro Ser Ser Ile Ala Ala Ala Ala Ile Leu Cys Val
225                 230                 235                 240

Ala Asn Glu Leu Pro Ser Leu Ser Ser Val Val Asn Pro His Glu Ser
                245                 250                 255

Pro Glu Thr Trp Cys Asp Gly Leu Ser Lys Glu Lys Ile Val Arg Cys
            260                 265                 270

Tyr Arg Leu Met Lys Ala Met Ala Ile Glu Asn Asn Arg Leu Asn Thr
        275                 280                 285

Pro Lys Val Ile Ala Lys Leu Arg Val Ser Val Arg Ala Ser Ser Thr
    290                 295                 300

Leu Thr Arg Pro Ser Asp Glu Ser Ser Ser Pro Cys Lys Arg Arg Lys
305                 310                 315                 320

Leu Ser Gly Tyr Ser Trp Val Gly Asp Glu Thr Ser Thr Ser Asn
                325                 330                 335

<210>  31
<211>  354
<212>  PRT
<213>  Nicotiana tabacum

<400>  31
Met Ala Ala Asp Asn Ile Tyr Asp Phe Val Ala Ser Asn Leu Leu Cys
  1               5                  10                  15

Thr Glu Thr Lys Ser Leu Cys Phe Asp Asp Val Asp Ser Leu Thr Ile
             20                  25                  30

Ser Gln Gln Asn Ile Glu Thr Lys Ser Lys Asp Leu Ser Phe Asn Asn
         35                  40                  45

Gly Ile Arg Ser Glu Pro Leu Ile Asp Leu Pro Ser Leu Ser Glu Glu
     50                  55                  60

Cys Leu Ser Phe Met Val Gln Arg Glu Met Glu Phe Leu Pro Lys Asp
 65                  70                  75                  80

Asp Tyr Val Glu Arg Leu Arg Ser Gly Asp Leu Asp Leu Ser Val Arg
                 85                  90                  95

Lys Glu Ala Leu Asp Trp Ile Leu Lys Ala His Met His Tyr Gly Phe
            100                 105                 110

Gly Glu Leu Ser Phe Cys Leu Ser Ile Asn Tyr Leu Asp Arg Phe Leu
        115                 120                 125

Ser Leu Tyr Glu Leu Pro Arg Ser Lys Thr Trp Thr Val Gln Leu Leu
    130                 135                 140

Ala Val Ala Cys Leu Ser Leu Ala Ala Lys Met Glu Glu Ile Asn Val
145                 150                 155                 160

Pro Leu Thr Val Asp Leu Gln Val Gly Asp Pro Lys Phe Val Phe Glu
                165                 170                 175

Gly Lys Thr Ile Gln Arg Met Glu Leu Leu Val Leu Ser Thr Leu Lys
            180                 185                 190

Trp Arg Met Gln Ala Tyr Thr Pro Tyr Thr Phe Ile Asp Tyr Phe Met
        195                 200                 205

Arg Lys Met Asn Gly Asp Gln Ile Pro Ser Arg Pro Leu Ile Ser Gly
    210                 215                 220

Ser Met Gln Leu Ile Leu Ser Ile Ile Arg Ser Ile Asp Phe Leu Glu
225                 230                 235                 240

Phe Arg Ser Ser Glu Ile Ala Ala Ser Val Ala Met Ser Val Ser Gly
                245                 250                 255

Glu Ile Gln Ala Lys Asp Ile Asp Lys Ala Met Pro Cys Phe Phe Ile
            260                 265                 270

His Leu Asp Lys Gly Arg Val Gln Lys Cys Val Glu Leu Ile Gln Asp
        275                 280                 285

Leu Thr Thr Ala Thr Ile Thr Thr Ala Ala Ala Ala Ser Leu Val Pro
    290                 295                 300

Gln Ser Pro Ile Gly Val Leu Glu Ala Ala Ala Cys Leu Ser Tyr Lys
305                 310                 315                 320

Ser Gly Asp Glu Arg Thr Val Gly Ser Cys Thr Thr Ser Ser His Thr
                325                 330                 335

Lys Arg Arg Lys Leu Asp Thr Ser Ser Leu Glu His Gly Thr Ser Glu
            340                 345                 350

Lys Leu

<210> 32
<211> 373
<212> PRT
<213> Nicotiana tabacum

<400> 32
Met Ala Ile Glu His Asn Glu Gln Gln Glu Leu Ser Gln Ser Phe Leu
1               5                   10                  15

Leu Asp Ala Leu Tyr Cys Glu Glu Glu Glu Glu Lys Trp Gly Asp Leu
            20                  25                  30

Val Asp Asp Glu Thr Ile Ile Thr Pro Leu Ser Ser Glu Val Thr Thr
        35                  40                  45

Thr Thr Thr Thr Thr Thr Lys Pro Asn Ser Leu Leu Pro Leu Leu Leu
    50                  55                  60

Leu Glu Gln Asp Leu Phe Trp Glu Asp Glu Glu Leu Leu Ser Leu Phe
65                  70                  75                  80

Ser Lys Glu Lys Glu Thr His Cys Trp Phe Asn Ser Phe Gln Asp Asp
                85                  90                  95

Ser Leu Leu Cys Ser Ala Arg Val Asp Ser Val Glu Trp Ile Leu Lys
            100                 105                 110

Val Asn Gly Tyr Tyr Gly Phe Ser Ala Leu Thr Ala Val Leu Ala Ile
        115                 120                 125

Asn Tyr Phe Asp Arg Phe Leu Thr Ser Leu His Tyr Gln Lys Asp Lys
    130                 135                 140

Pro Trp Met Ile Gln Leu Ala Ala Val Thr Cys Leu Ser Leu Ala Ala
145                 150                 155                 160

Lys Val Glu Glu Thr Gln Val Pro Leu Leu Leu Asp Phe Gln Val Glu
                165                 170                 175

Asp Ala Lys Tyr Val Phe Glu Ala Lys Thr Ile Gln Arg Met Glu Leu
            180                 185                 190

Leu Val Leu Ser Ser Leu Lys Trp Arg Met Asn Pro Val Thr Pro Leu
        195                 200                 205

Ser Phe Leu Asp His Ile Ile Arg Arg Leu Gly Leu Arg Asn Asn Ile
    210                 215                 220

His Trp Glu Phe Leu Arg Arg Cys Glu Asn Leu Leu Leu Ser Ile Met
225                 230                 235                 240

Ala Asp Cys Arg Phe Val Arg Tyr Met Pro Ser Val Leu Ala Thr Ala
                245                 250                 255

Ile Met Leu His Val Ile His Gln Val Glu Pro Cys Asn Ser Val Asp
            260                 265                 270

Tyr Gln Asn Gln Leu Leu Gly Val Leu Lys Ile Asn Lys Glu Lys Val
        275                 280                 285

Asn Asn Cys Phe Glu Leu Ile Ser Glu Val Cys Ser Lys Pro Ile Ser
    290                 295                 300

His Lys Arg Lys Tyr Glu Asn Pro Ser His Ser Pro Ser Gly Val Ile
305                 310                 315                 320

Asp Pro Ile Tyr Ser Ser Glu Ser Ser Asn Asp Ser Trp Asp Leu Glu
                325                 330                 335

Ser Thr Ser Ser Tyr Phe Pro Val Phe Lys Lys Ser Arg Val Gln Glu
            340                 345                 350

Gln Gln Met Lys Leu Ala Ser Ser Ile Ser Arg Val Phe Val Glu Ala
        355                 360                 365

Val Gly Ser Pro His
    370

## Description

This application claims the benefit of U.S. Provisional Application No. 60/078,948, filed March 23, 1998.

### FIELD OF THE INVENTION

This invention is in the field of plant molecular biology. More specifically, this invention pertains to nucleic acid fragments encoding cyclin proteins in plants and seeds.

### BACKGROUND OF THE INVENTION

Cells divide by duplicating their chromosomes and segregating one copy of each duplicated chromosome, as well as providing essential organelles, to each of two daughter cells. Regulation of cell division is critical for the normal development of multicellular organisms. A cell that is destined to grow and divide must pass through specific phases of a cell cycle: G₁, S (period of DNA synthesis), G₂, and M (mitosis). Studies have shown that cell division is controlled via the regulation of two critical events during the cell cycle: initiation of DNA synthesis and the initiation of mitosis. Several kinase proteins control cell cycle progression through these events. These protein kinases are heterodimeric proteins, having a cyclin-dependent kinase (Cdks) subunit and a cyclin subunit that provides the regulatory specificity to the heterodimeric protein. These heterodimeric proteins regulate cell cycle by interacting with proteins involved in the initiation of DNA synthesis and mitosis and phosphorylating them at specific regulatory sites, activating some and inactivating others. The cyclin subunit concentration varies in phase with cell cycle while the concentration of the Cdks remain relatively constant throughout the cell cycle.

In eukaryotic cells several different cyclin proteins have been identified that regulate cell cycle. Cyclins D (delta) and E appear to function during G₁ phase to regulate progression to S phase (Soni, B. et al. (1995) *Plant Cell 7(1)*:85-103; Sorrell, D.A. et al. (1999) *Plant Physiol. 199*:343-351). Cyclin A functions during S and G₂ phases to regulate DNA synthesis and cell cycle progression into mitosis and Cyclin B functions only during G₂ phase to control cell cycle entry into mitosis (Kouchi, H. et al. (1995) *Plant Cell 7(8)*:143-1155). Because the cyclin subunit provides specificity for controlling the cell cycle they are obvious targets for manipulating cell-cycle regulation in eukaryotes. There is a great deal of interest in identifying the genes that encode cyclins in plants. These genes may be used to express cyclins in plant cells to enhance cell tissue culture growth. Accordingly, the availability of nucleic acid sequences encoding all or a portion of cyclins would facilitate studies to better understand cell cycle in plants, provide genetic tools to enhance cell growth in tissue culture, increase the efficiency of gene transfer and help provide more stable transformations. Cyclins may also provide targets to facilitate design and/or identification of inhibitors of cyclins that may be useful as herbicides.

### SUMMARY OF THE INVENTION

The instant invention relates to isolated nucleic acid fragments encoding cyclin proteins. Specifically, this invention concerns an isolated nucleic acid fragment encoding a cyclin A, cyclin delta-1, cyclin delta-2 or cyclin delta-3. In addition, this invention relates to a nucleic acid fragment that is complementary to the nucleic acid fragment encoding cyclin A, cyclin delta-1, cyclin delta-2 or cyclin delta-3.

An additional embodiment of the instant invention pertains to a polypeptide encoding all or a substantial portion of a cyclin protein selected from the group consisting of cyclin A, cyclin delta-1, cyclin delta-2 or cyclin delta-3.

In another embodiment, the instant invention relates to a chimeric gene encoding a cyclin A, cyclin delta-1, cyclin delta-2 or cyclin delta-3, or to a chimeric gene that comprises a nucleic acid fragment that is complementary to a nucleic acid fragment encoding a cyclin A, cyclin delta-1, cyclin delta-2 or cyclin delta-3, operably linked to suitable regulatory sequences, wherein expression of the chimeric gene results in production of levels of the encoded protein in a transformed host cell that is altered (i.e., increased or decreased) from the level produced in an untransformed host cell.

In a further embodiment, the instant invention concerns a transformed host cell comprising in its genome a chimeric gene encoding a cyclin A, cyclin delta-1, cyclin delta-2 or cyclin delta-3, operably linked to suitable regulatory sequences. Expression of the chimeric gene results in production of altered levels of the encoded protein in the transformed host cell. The transformed host cell can be of eukaryotic or prokaryotic origin, and include cells derived from higher plants and microorganisms. The invention also includes transformed plants that arise from transformed host cells of higher plants, and seeds derived from such transformed plants.

An additional embodiment of the instant invention concerns a method of altering the level of expression of a cyclin A, cyclin delta-1, cyclin delta-2 or cyclin delta-3 in a transformed host cell comprising: a) transforming a host cell with a chimeric gene comprising a nucleic acid fragment encoding a cyclin A, cyclin delta-1, cyclin delta-2 or cyclin delta-3; and b) growing the transformed host cell under conditions that are suitable for expression of the chimeric gene wherein expression of the chimeric gene results in production of altered levels of cyclin A, cyclin delta-1, cyclin delta-2 or cyclin delta-3 in the transformed host cell.

An addition embodiment of the instant invention concerns a method for obtaining a nucleic acid fragment encoding all or a substantial portion of an amino acid sequence encoding a cyclin A, cyclin delta-1, cyclin delta-2 or cyclin delta-3.

A further embodiment of the instant invention is a method for evaluating at least one compound for its ability to inhibit the activity of a cyclin A, cyclin delta-1, cyclin delta-2 or cyclin delta-3, the method comprising the steps of: (a) transforming a host cell with a chimeric gene comprising a nucleic acid fragment encoding a cyclin A, cyclin delta-1, cyclin delta-2 or cyclin delta-3, operably linked to suitable regulatory sequences; (b) growing the transformed host cell under conditions that are suitable for expression of the chimeric gene wherein expression of the chimeric gene results in production of cyclin A, cyclin delta-1, cyclin delta-2 or cyclin delta-3 in the transformed host cell; (c) optionally purifying the cyclin A, cyclin delta-1, cyclin delta-2 or cyclin delta-3 expressed by the transformed host cell; (d) treating the cyclin A, cyclin delta-1, cyclin delta-2 or cyclin delta-3 with a compound to be tested; and (e) comparing the activity of the cyclin A, cyclin delta-1, cyclin delta-2 or cyclin delta-3 that has been treated with a test compound to the activity of an untreated cyclin A, cyclin delta-1, cyclin delta-2 or cyclin delta-3, thereby selecting compounds with potential for inhibitory activity.

### BRIEF DESCRIPTION OF THE DRAWINGS AND SEQUENCE DESCRIPTIONS

The invention can be more fully understood from the following detailed description and the accompanying drawings and Sequence Listing which form a part of this application.
Figure 1 shows a comparison of the amino acid sequence set forth in SEQ ID NO:2 and the *Catharanthus roseus* sequence, SEQ ID NO:29.
Figure 2 shows a comparison of the amino acid sequences set forth in SEQ ID NOs:8, 10, 12 and 14 and the *A. thaliana* cyclin delta-1 sequence, SEQ ID NO:30.
Figure 3 shows a comparison of the amino acid sequences set forth in SEQ ID NOs:18 and 22 and the *Nicotina tabacum* sequence, SEQ ID NO:31.
Figure 4 shows a comparison of the amino acid sequences set forth in SEQ ID NO:24 and the *Nicotiana tabacum* sequence, SEQ ID NO:32.

The following sequence descriptions and Sequence Listing attached hereto comply with the rules governing nucleotide and/or amino acid sequence disclosures in patent applications as set forth in 37 C.F.R. §1.821-1.825.

SEQ ID NO:1 is the nucleotide sequence comprising a contig assembled from the cDNA inserts in clones p0072.comfl88rb and cen3n.pk0208.h3 encoding a portion of a corn cyclin A protein.

SEQ ID NO:2 is the deduced amino acid sequence of a portion of a cyclin A protein derived from the nucleotide sequence of SEQ ID NO:1.

SEQ ID NO:3 is the nucleotide sequence comprising a portion of the cDNA insert in clone srm.pk0017.h9 encoding a portion of a soybean cyclin A protein.

SEQ ID NO:4 is the deduced amino acid sequence of a portion of a cyclin A protein derived from the nucleotide sequence of SEQ ID NO:3.

SEQ ID NO:5 is the nucleotide sequence comprising a contig assembled from the cDNA inserts in clones wlmk1.pk0009.b7 and wrl.pk0093.fl1 encoding a portion of a wheat cyclin A protein.

SEQ ID NO:6 is the deduced amino acid sequence of a portion of a cyclin A protein derived from the nucleotide sequence of SEQ ID NO:1.

SEQ ID NO:7 is the nucleotide sequence comprising a contig assembled from the cDNA inserts in clones p0128.cpiad46rb, p0116.cesaf50r and p0098.cdfae90r encoding a portion of a corn cyclin delta-1 protein.

SEQ ID NO:8 is the deduced amino acid sequence of a portion of a cyclin delta-1 protein derived from the nucleotide sequence of SEQ ID NO:7.

SEQ ID NO:9 is the nucleotide sequence comprising a portion of the cDNA insert in clone r10n.pk0031.e6 encoding a portion of a rice cyclin delta-1 protein.

SEQ ID NO:10 is the deduced amino acid sequence of a portion of a cyclin delta-1 protein derived from the nucleotide sequence of SEQ ID NO:9.

SEQ ID NO:11 is the nucleotide sequence comprising a contig assembled from the cDNA inserts in clones sah1c.pk003.i7 and sr1.pk0001.g5 encoding an entire soybean cyclin delta-1 protein.

SEQ ID NO:12 is the deduced amino acid sequence of a cyclin delta-1 protein derived from the nucleotide sequence of SEQ ID NO:11.

SEQ ID NO:13 is the nucleotide sequence comprising a portion of the cDNA insert in clone se6.pk0028.f11 encoding a portion of a soybean cyclin delta-1 protein.

SEQ ID NO:14 is the deduced amino acid sequence of a portion of a cyclin delta-1 protein derived from the nucleotide sequence of SEQ ID NO:13.

SEQ ID NO:15 is the nucleotide sequence comprising a portion of the cDNA insert in clone wle1n.pk0036.e2 encoding a portion of a wheat cyclin delta-1 protein.

SEQ ID NO:16 is the deduced amino acid sequence of a portion of a cyclin delta-1 protein derived from the nucleotide sequence of SEQ ID NO:15.

SEQ ID NO:17 is the nucleotide sequence comprising a portion of the cDNA insert in clone ceb5.pk0049.h5 encoding a portion of a corn cyclin delta-2 protein.

SEQ ID NO:18 is the deduced amino acid sequence of a portion of a cyclin delta-2 protein derived from the nucleotide sequence of SEQ ID NO: 17.

SEQ ID NO:19 is the nucleotide sequence comprising a portion of the cDNA insert in clone r10n.pk091.m14 encoding a portion of a rice cyclin delta-2 protein.

SEQ ID NO:20 is the deduced amino acid sequence of a portion of a cyclin delta-2 protein derived from the nucleotide sequence of SEQ ID NO:19.

SEQ ID NO:21 is the nucleotide sequence comprising a portion of the cDNA insert in clone wreln.pk0104.c1 encoding a portion of a wheat cyclin delta-2 protein.

SEQ ID NO:22 is the deduced amino acid sequence of a portion of a cyclin delta-2 protein derived from the nucleotide sequence of SEQ ID NO:21.

SEQ ID NO:23 is the nucleotide sequence comprising a portion of the cDNA insert in clone crln.pk0185.g7 encoding a portion of a corn cyclin delta-3 protein.

SEQ ID NO:24 is the deduced amino acid sequence of a portion of a cyclin delta-3 protein derived from the nucleotide sequence of SEQ ID NO:23.

SEQ ID NO:25 is the nucleotide sequence comprising a contig assembled from the cDNA inserts in clones ses9c.pk002.h24, sr1.pk0011.d11, scb1c.pk002.c13 encoding a portion of a soybean cyclin delta-3 protein.

SEQ ID NO:26 is the deduced amino acid sequence of a portion of a cyclin delta-3 protein derived from the nucleotide sequence of SEQ ID NO:25.

SEQ ID NO:27 is the nucleotide sequence comprising a contig assembled from the cDNA inserts in clones sfl1.pk0001.a8 and sre.pk0035.b5 encoding a portion of a soybean cyclin delta-3 protein.

SEQ ID NO:28 is the deduced amino acid sequence of a portion of a cyclin delta-3 protein derived from the nucleotide sequence of SEQ ID NO:27.

SEQ ID NO:29 is the amino acid sequence of a *Catharanthus roseus* (NCBI Identifier No. gi 2190259) cyclin A protein.

SEQ ID NO:30 is the amino acid sequence of an *Arabidopsis thaliana* (NCBI Identifier No. gi 3915635) cyclin delta-1 protein.

SEQ ID NO:31 is the amino acid sequence of a *Nicotiana tabacum* (NCBI Identifier No. gi 4160298) cyclin delta-2 protein.

SEQ ID NO:32 is the amino acid sequence of a *Nicotiana tabacum* (NCBI Identifier No. gi 4160300) cyclin delta-3 protein.

The Sequence Listing contains the one letter code for nucleotide sequence characters and the three letter codes for amino acids as defined in conformity with the IUPAC-IUBMB standards described in *Nucleic Acids Research 13*:3021-3030 (1985) and in the *Biochemical Journal 219 (No. 2)*:345-373 (1984) which are herein incorporated by reference. The symbols and format used for nucleotide and amino acid sequence data comply with the rules set forth in 37 C.F.R. §1.822.

### DETAILED DESCRIPTION OF THE INVENTION

In the context of this disclosure, a number of terms shall be utilized. As used herein, an "isolated nucleic acid fragment" is a polymer of RNA or DNA that is single- or double-stranded, optionally containing synthetic, non-natural or altered nucleotide bases. An isolated nucleic acid fragment in the form of a polymer of DNA may be comprised of one or more segments of cDNA, genomic DNA or synthetic DNA. As used herein, "contig" refers to an assemblage of overlapping nucleic acid sequences to form one contiguous nucleotide sequence. For example, several DNA sequences can be compared and aligned to identify common or overlapping regions. The individual sequences can then be assembled into a single contiguous nucleotide sequence.

As used herein, "substantially similar" refers to nucleic acid fragments wherein changes in one or more nucleotide bases results in substitution of one or more amino acids, but do not affect the functional properties of the protein encoded by the DNA sequence.

"Substantially similar" also refers to nucleic acid fragments wherein changes in one or more nucleotide bases does not affect the ability of the nucleic acid fragment to mediate alteration of gene expression by antisense or co-suppression technology. "Substantially similar" also refers to modifications of the nucleic acid fragments of the instant invention such as deletion or insertion of one or more nucleotides that do not substantially affect the functional properties of the resulting transcript vis-à-vis the ability to mediate alteration of gene expression by antisense or co-suppression technology or alteration of the functional properties of the resulting protein molecule. It is therefore understood that the invention encompasses more than the specific exemplary sequences.

For example, it is well known in the art that antisense suppression and co-suppression of gene expression may be accomplished using nucleic acid fragments representing less than the entire coding region of a gene, and by nucleic acid fragments that do not share 100% sequence identity with the gene to be suppressed. Moreover, alterations in a gene which result in the production of a chemically equivalent amino acid at a given site, but do not effect the functional properties of the encoded protein, are well known in the art. Thus, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also be expected to produce a functionally equivalent product. Nucleotide changes which result in alteration of the N-terminal and C-terminal portions of the protein molecule would also not be expected to alter the activity of the protein. Each of the proposed modifications is well within the routine skill in the art, as is determination of retention of biological activity of the encoded products.

Moreover, substantially similar nucleic acid fragments may also be characterized by their ability to hybridize, under stringent conditions (0.1X SSC, 0.1% SDS, 65°C), with the nucleic acid fragments disclosed herein.

Substantially similar nucleic acid fragments of the instant invention may also be characterized by the percent similarity of the amino acid sequences that they encode to the amino acid sequences disclosed herein, as determined by algorithms commonly employed by those skilled in this art. Preferred are those nucleic acid fragments whose nucleotide sequences encode amino acid sequences that are 80% similar to the amino acid sequences reported herein. More preferred nucleic acid fragments encode amino acid sequences that are 90% similar to the amino acid sequences reported herein. Most preferred are nucleic acid fragments that encode amino acid sequences that are 95% similar to the amino acid sequences reported herein. Sequence alignments and percent similarity calculations were performed using the Megalign program of the LASARGENE bioinformatics computing suite (DNASTAR Inc., Madison, WI). Multiple alignment of the sequences was performed using the Clustal method of alignment (Higgins, D. G. and Sharp, P. M. (1989) *CABIOS*. 5:151-153) with the default parameters (GAP PENALTY=10, GAP LENGTH PENALTY=10). Default parameters for pairwise alignments using the Clustal method were KTUPLE 1, GAP PENALTY=3, WINDOW=5 and DIAGONALS SAVED=5.

A "substantial portion" of an amino acid or nucleotide sequence comprises enough of the amino acid sequence of a polypeptide or the nucleotide sequence of a gene to afford putative identification of that polypeptide or gene, either by manual evaluation of the sequence by one skilled in the art, or by computer-automated sequence comparison and identification using algorithms such as BLAST (Basic Local Alignment Search Tool; Altschul, S. F., et al., (1993) *J. Mol. Biol. 215*:403-410; see also www.ncbi.nlm.nih.gov/BLAST/). In general, a sequence often or more contiguous amino acids or thirty or more nucleotides is necessary in order to putatively identify a polypeptide or nucleic acid sequence as homologous to a known protein or gene. Moreover, with respect to nucleotide sequences, gene specific oligonucleotide probes comprising 20-30 contiguous nucleotides may be used in sequence-dependent methods of gene identification (e.g., Southern hybridization) and isolation (e.g., *in situ* hybridization of bacterial colonies or bacteriophage plaques). In addition, short oligonucleotides of 12-15 bases may be used as amplification primers in PCR in order to obtain a particular nucleic acid fragment comprising the primers. Accordingly, a "substantial portion" of a nucleotide sequence comprises enough of the sequence to afford specific identification and/or isolation of a nucleic acid fragment comprising the sequence. The instant specification teaches partial or complete amino acid and nucleotide sequences encoding one or more particular plant proteins. The skilled artisan, having the benefit of the sequences as reported herein, may now use all or a substantial portion of the disclosed sequences for purposes known to those skilled in this art. Accordingly, the instant invention comprises the complete sequences as reported in the accompanying Sequence Listing, as well as substantial portions of those sequences as defined above.

"Codon degeneracy" refers to divergence in the genetic code permitting variation of the nucleotide sequence without effecting the amino acid sequence of an encoded polypeptide. Accordingly, the instant invention relates to any nucleic acid fragment that encodes all or a substantial portion of the amino acid sequence encoding the cyclin A, cyclin delta-1, cyclin delta-2 or cyclin delta-3 proteins as set forth in SEQ ID NOs:2, 4, 6, 8, 10, 12, 14, 16, 18, 20, 22, 24, 26 and 28. The skilled artisan is well aware of the "codon-bias" exhibited by a specific host cell in usage of nucleotide codons to specify a given amino acid. Therefore, when synthesizing a gene for improved expression in a host cell, it is desirable to design the gene such that its frequency of codon usage approaches the frequency of preferred codon usage of the host cell.

"Synthetic genes" can be assembled from oligonucleotide building blocks that are chemically synthesized using procedures known to those skilled in the art. These building blocks are ligated and annealed to form gene segments which are then enzymatically assembled to construct the entire gene. "Chemically synthesized", as related to a sequence of DNA, means that the component nucleotides were assembled *in vitro.* Manual chemical synthesis of DNA may be accomplished using well established procedures, or automated chemical synthesis can be performed using one of a number of commercially available machines. Accordingly, the genes can be tailored for optimal gene expression based on optimization of nucleotide sequence to reflect the codon bias of the host cell. The skilled artisan appreciates the likelihood of successful gene expression if codon usage is biased towards those codons favored by the host. Determination of preferred codons can be based on a survey of genes derived from the host cell where sequence information is available.

"Gene" refers to a nucleic acid fragment that expresses a specific protein, including regulatory sequences preceding (5' non-coding sequences) and following (3' non-coding sequences) the coding sequence. "Native gene" refers to a gene as found in nature with its own regulatory sequences. "Chimeric gene" refers any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. "Endogenous gene" refers to a native gene in its natural location in the genome of an organism. A "foreign" gene refers to a gene not normally found in the host organism, but that is introduced into the host organism by gene transfer. Foreign genes can comprise native genes inserted into a non-native organism, or chimeric genes. A "transgene" is a gene that has been introduced into the genome by a transformation procedure.

"Coding sequence" refers to a DNA sequence that codes for a specific amino acid sequence. "Regulatory sequences" refer to nucleotide sequences located upstream (5' non-coding sequences), within, or downstream (3' non-coding sequences) of a coding sequence, and which influence the transcription, RNA processing or stability, or translation of the associated coding sequence. Regulatory sequences may include promoters, translation leader sequences, introns, and polyadenylation recognition sequences.

"Promoter" refers to a DNA sequence capable of controlling the expression of a coding sequence or functional RNA. In general, a coding sequence is located 3' to a promoter sequence. The promoter sequence consists of proximal and more distal upstream elements, the latter elements often referred to as enhancers. Accordingly, an "enhancer" is a DNA sequence which can stimulate promoter activity and may be an innate element of the promoter or a heterologous element inserted to enhance the level or tissue-specificity of a promoter. Promoters may be derived in their entirety from a native gene, or be composed of different elements derived from different promoters found in nature, or even comprise synthetic DNA segments. It is understood by those skilled in the art that different promoters may direct the expression of a gene in different tissues or cell types, or at different stages of development, or in response to different environmental conditions. Promoters which cause a gene to be expressed in most cell types at most times are commonly referred to as "constitutive promoters". New promoters of various types useful in plant cells are constantly being discovered; numerous examples may be found in the compilation by Okamuro and Goldberg, (1989) *Biochemistry of Plants 15*:1-82. It is further recognized that since in most cases the exact boundaries of regulatory sequences have not been completely defined, DNA fragments of different lengths may have identical promoter activity.

The "translation leader sequence" refers to a DNA sequence located between the promoter sequence of a gene and the coding sequence. The translation leader sequence is present in the fully processed mRNA upstream of the translation start sequence. The translation leader sequence may affect processing of the primary transcript to mRNA, mRNA stability or translation efficiency. Examples of translation leader sequences have been described (Turner, R. and Foster, G. D. (1995) *Molecular Biotechnology 3*:225).

The "3' non-coding sequences" refer to DNA sequences located downstream of a coding sequence and include polyadenylation recognition sequences and other sequences encoding regulatory signals capable of affecting mRNA processing or gene expression. The polyadenylation signal is usually characterized by affecting the addition of polyadenylic acid tracts to the 3' end of the mRNA precursor. The use of different 3' non-coding sequences is exemplified by Ingelbrecht et al., (1989) *Plant Cell 1*:671-680.

"RNA transcript" refers to the product resulting from RNA polymerase-cataiyzed transcription of a DNA sequence. When the RNA transcript is a perfect complementary copy of the DNA sequence, it is referred to as the primary transcript or it may be a RNA sequence derived from posttranscriptional processing of the primary transcript and is referred to as the mature RNA. "Messenger RNA (mRNA)" refers to the RNA that is without introns and that can be translated into protein by the cell. "cDNA" refers to a double-stranded DNA that is complementary to and derived from mRNA. "Sense" RNA refers to RNA transcript that includes the mRNA and so can be translated into protein by the cell. "Antisense RNA" refers to a RNA transcript that is complementary to all or part of a target primary transcript or mRNA and that blocks the expression of a target gene (U.S. Pat. No. 5,107,065, incorporated herein by reference). The complementarity of an antisense RNA may be with any part of the specific gene transcript, i.e., at the 5' non-coding sequence, 3' non-coding sequence, introns, or the coding sequence. "Functional RNA" refers to sense RNA, antisense RNA, ribozyme RNA, or other RNA that may not be translated but yet has an effect on cellular processes.

The term "operably linked" refers to the association of nucleic acid sequences on a single nucleic acid fragment so that the function of one is affected by the other. For example, a promoter is operably linked with a coding sequence when it is capable of affecting the expression of that coding sequence (i.e., that the coding sequence is under the transcriptional control of the promoter). Coding sequences can be operably linked to regulatory sequences in sense or antisense orientation.

The term "expression", as used herein, refers to the transcription and stable accumulation of sense (mRNA) or antisense RNA derived from the nucleic acid fragment of the invention. Expression may also refer to translation of mRNA into a polypeptide. "Antisense inhibition" refers to the production of antisense RNA transcripts capable of suppressing the expression of the target protein. "Overexpression" refers to the production of a gene product in transgenic organisms that exceeds levels of production in normal or non-transformed organisms. "Co-suppression" refers to the production of sense RNA transcripts capable of suppressing the expression of identical or substantially similar foreign or endogenous genes (U.S. Pat. No. 5,231,020, incorporated herein by reference).

"Altered levels" refers to the production of gene product(s) in transgenic organisms in amounts or proportions that differ from that of normal or non-transformed organisms.

"Mature" protein refers to a post-translationally processed polypeptide; i.e., one from which any pre- or propeptides present in the primary translation product have been removed. "Precursor" protein refers to the primary product of translation of mRNA; i.e., with pre- and propeptides still present. Pre- and propeptides may be but are not limited to intracellular localization signals.

A "chloroplast transit peptide" is an amino acid sequence which is translated in conjunction with a protein and directs the protein to the chloroplast or other plastid types present in the cell in which the protein is made. "Chloroplast transit sequence" refers to a nucleotide sequence that encodes a chloroplast transit peptide. A "signal peptide" is an amino acid sequence which is translated in conjunction with a protein and directs the protein to the secretory system (Chrispeels, J. J., (1991) *Ann. Rev. Plant Phys. Plant Mol. Biol. 42*:21-53). If the protein is to be directed to a vacuole, a vacuolar targeting signal *(supra)* can further be added, or if to the endoplasmic reticulum, an endoplasmic reticulum retention signal (*supra*) may be added. If the protein is to be directed to the nucleus, any signal peptide present should be removed and instead a nuclear localization signal included (Raikhel (1992) *Plant Phys.100*:1627-1632).

"Transformation" refers to the transfer of a nucleic acid fragment into the genome of a host organism, resulting in genetically stable inheritance. Host organisms containing the transformed nucleic acid fragments are referred to as "transgenic" organisms. Examples of methods of plant transformation include Agrobacterium-mediated transformation (De Blaere et al. (1987) *Meth. Enzymol. 143*:277) and particle-accelerated or "gene gun" transformation technology (Klein et al. (1987) *Nature (London) 327:70-73;* U.S. Pat. No. 4,945,050, incorporated herein by reference).

Standard recombinant DNA and molecular cloning techniques used herein are well known in the art and are described more fully in Sambrook, J., Fritsch, E. F. and Maniatis, T. *Molecular Cloning: A Laboratory Manual;* Cold Spring Harbor Laboratory Press: Cold Spring Harbor, 1989 (hereinafter "Maniatis").

Nucleic acid fragments encoding at least a portion of several cyclin proteins have been isolated and identified by comparison of random plant cDNA sequences to public databases containing nucleotide and protein sequences using the BLAST algorithms well known to those skilled in the art. Table 1 lists the proteins that are described herein, and the designation of the cDNA clones that comprise the nucleic acid fragments encoding these proteins.

**TABLE 1**

| Cyclin Proteins | | |
|---|---|---|
| Enzyme | Clone | Plant |
| Cyclin A | cen3n.pk0208.h3 | Corn |
| | p0072.comfl88rb | Corn |
| | srm.pk0017.h9 | Soybean |
| | wlmk1.pk0009.b7 | Wheat |
| | wr1.pk0093.f11 | Wheat |
| Cyclin delta-1 | p0098.cdfae90r | Corn |
| | p0116.cesaf50r | Corn |
| | p0128.cpiad46rb | Corn |
| | r10n.pk0031.e6 | Rice |
| | sah1c.pk003.i7 | Soybean |
| | sr1.pk0001.g5 | Soybeans |
| | se6.pk0028.f11 | Soybean |
| | wle1n.pk0036.e2 | Wheat |
| Cyclin delta-2 | ceb5.pk0049.h5 | Corn |
| | r10n.pk091.m14 | Rice |
| | wr1.pk0112.b2 | Wheat |
| | wre1n.pk0104.c1 | Wheat |
| Cyclin delta-3 | cr1n.pk0185.g7 | Corn |
| | ses9c.pk002.h24 | Soybean |
| | scb1c.pk002.c13 | Soybean |
| | sr1.pk0011.d11 | Soybean |
| | sfl1.pk0001.a8 | Soybean |
| | sre.pk0035.b5 | Soybean |

The nucleic acid fragments of the instant invention may be used to isolate cDNAs and genes encoding homologous proteins from the same or other plant species. Isolation of homologous genes using sequence-dependent protocols is well known in the art. Examples of sequence-dependent protocols include, but are not limited to, methods of nucleic acid hybridization, and methods of DNA and RNA amplification as exemplified by various uses of nucleic acid amplification technologies (e.g., polymerase chain reaction, ligase chain reaction).

For example, genes encoding other cyclin A, cyclin delta-1, cyclin delta-2 or cyclin delta-3 proteins, either as cDNAs or genomic DNAs, could be isolated directly by using all or a portion of the instant nucleic acid fragments as DNA hybridization probes to screen libraries from any desired plant employing methodology well known to those skilled in the art. Specific oligonucleotide probes based upon the instant nucleic acid sequences can be designed and synthesized by methods known in the art (Maniatis). Moreover, the entire sequences can be used directly to synthesize DNA probes by methods known to the skilled artisan such as random primer DNA labeling, nick translation, or end-labeling techniques, or RNA probes using available *in vitro* transcription systems. In addition, specific primers can be designed and used to amplify a part or all of the instant sequences. The resulting amplification products can be labeled directly during amplification reactions or labeled after amplification reactions, and used as probes to isolate full length cDNA or genomic fragments under conditions of appropriate stringency.

In addition, two short segments of the instant nucleic acid fragments may be used in polymerase chain reaction protocols to amplify longer nucleic acid fragments encoding homologous genes from DNA or RNA. The polymerase chain reaction may also be performed on a library of cloned nucleic acid fragments wherein the sequence of one primer is derived from the instant nucleic acid fragments, and the sequence of the other primer takes advantage of the presence of the polyadenylic acid tracts to the 3' end of the mRNA precursor encoding plant genes. Alternatively, the second primer sequence may be based upon sequences derived from the cloning vector. For example, the skilled artisan can follow the RACE protocol (Frohman et al., (1988) *PNAS USA 85*:8998) to generate cDNAs by using PCR to amplify copies of the region between a single point in the transcript and the 3' or 5' end. Primers oriented in the 3' and 5' directions can be designed from the instant sequences. Using commercially available 3' RACE or 5' RACE systems (BRL), specific 3' or 5' cDNA fragments can be isolated (Ohara et al., (1989) *PNAS USA 86*:5673; Loh et al., (1989) *Science 243*:217). Products generated by the 3' and 5' RACE procedures can be combined to generate full-length cDNAs (Frohman, M. A. and Martin, G. R., (1989) *Techniques 1*:165).

Availability of the instant nucleotide and deduced amino acid sequences facilitates immunological screening of cDNA expression libraries. Synthetic peptides representing portions of the instant amino acid sequences may be synthesized. These peptides can be used to immunize animals to produce polyclonal or monoclonal antibodies with specificity for peptides or proteins comprising the amino acid sequences. These antibodies can be then be used to screen cDNA expression libraries to isolate full-length cDNA clones of interest (Lerner, R. A. (1984) *Adv. Immunol. 36*:1; Maniatis).

The nucleic acid fragments of the instant invention may be used to create transgenic plants in which the disclosed cyclin A, cyclin delta-1, cyclin delta-2 or cyclin delta-3 are present at higher or lower levels than normal or in cell types or developmental stages in which they are not normally found. This would have the effect of altering the regulation of cell division in those cells. The nucleic acid fragments may be used to express cyclins in plant cells to enhance cell tissue culture growth. Accordingly, the availability of nucleic acid sequences encoding all or a portion of cyclins would facilitate studies to better understand cell cycle in plants, provide genetic tools to enhance cell growth in tissue culture, increase the efficiency of gene transfer and help provide more stable transformations. Cyclins may also provide targets to facilitate design and/or identification of inhibitors of cyclins that may be useful as herbicides.

Overexpression of the cyclin A, cyclin delta-1, cyclin delta-2 or cyclin delta-3 proteins of the instant invention may be accomplished by first constructing a chimeric gene in which the coding region is operably linked to a promoter capable of directing expression of a gene in the desired tissues at the desired stage of development. For reasons of convenience, the chimeric gene may comprise promoter sequences and translation leader sequences derived from the same genes. 3' Non-coding sequences encoding transcription termination signals may also be provided. The instant chimeric gene may also comprise one or more introns in order to facilitate gene expression.

Plasmid vectors comprising the instant chimeric gene can then constructed. The choice of plasmid vector is dependent upon the method that will be used to transform host plants. The skilled artisan is well aware of the genetic elements that must be present on the plasmid vector in order to successfully transform, select and propagate host cells containing the chimeric gene. The skilled artisan will also recognize that different independent transformation events will result in different levels and patterns of expression (Jones et al., (1985) *EMBO J. 4*:2411-2418; De Almeida et al., (1989) *Mol. Gen. Genetics 218*:78-86), and thus that multiple events must be screened in order to obtain lines displaying the desired expression level and pattern. Such screening may be accomplished by Southern analysis of DNA, Northern analysis of mRNA expression, Western analysis of protein expression, or phenotypic analysis.

For some applications it may be useful to direct the instant cyclin proteins to different cellular compartments, or to facilitate its secretion from the cell. It is thus envisioned that the chimeric gene described above may be further supplemented by altering the coding sequence to encode cyclin A, cyclin delta-1, cyclin delta-2 or cyclin delta-3 with appropriate intracellular targeting sequences such as transit sequences (Keegstra, K. (1989) *Cell 56:*247-253), signal sequences or sequences encoding endoplasmic reticulum localization (Chrispeels, J. J., (1991) *Ann. Rev. Plant Phys. Plant Mol. Biol. 42*:21-53), or nuclear localization signals (Raikhel, N. (1992) *Plant Phys.100*:1627-1632) added and/or with targeting sequences that are already present removed. While the references cited give examples of each of these, the list is not exhaustive and more targeting signals of utility may be discovered in the future.

It may also be desirable to reduce or eliminate expression of genes encoding cyclin A, cyclin delta-1, cyclin delta-2 or cyclin delta-3 in plants for some applications. In order to accomplish this, a chimeric gene designed for co-suppression of the instant cyclin proteins can be constructed by linking a gene or gene fragment encoding an cyclin A, cyclin delta-1, cyclin delta-2 or cyclin delta-3 to plant promoter sequences. Alternatively, a chimeric gene designed to express antisense RNA for all or part of the instant nucleic acid fragment can be constructed by linking the gene or gene fragment in reverse orientation to plant promoter sequences. Either the co-suppression or antisense chimeric genes could be introduced into plants via transformation wherein expression of the corresponding endogenous genes are reduced or eliminated.

The instant cyclin A, cyclin delta-1, cyclin delta-2 or cyclin delta-3 proteins (or portions thereof) may be produced in heterologous host cells, particularly in the cells of microbial hosts, and can be used to prepare antibodies to the these proteins by methods well known to those skilled in the art. The antibodies are useful for detecting cyclin A, cyclin delta-1, cyclin delta-2 and cyclin delta-3 *in situ* in cells or *in vitro* in cell extracts. Preferred heterologous host cells for production of the instant cyclin A, cyclin delta-1, cyclin delta-2 or cyclin delta-3 are microbial hosts. Microbial expression systems and expression vectors containing regulatory sequences that direct high level expression of foreign proteins are well known to those skilled in the art. Any of these could be used to construct a chimeric gene for production of the instant cyclin A, cyclin delta-1, cyclin delta-2 or cyclin delta-3 proteins. This chimeric gene could then be introduced into appropriate microorganisms via transformation to provide high level expression of the encoded cyclin proteins. An example of a vector for high level expression of the instant cyclin A, cyclin delta-1, cyclin delta-2 or cyclin delta-3 in a bacterial host is provided (Example 9).

Additionally, the instant cyclin A, cyclin delta-1, cyclin delta-2 or cyclin delta-3 can be used as a targets to facilitate design and/or identification of inhibitors of those enzymes that may be useful as herbicides. This is desirable because the delta-1 cyclin, delta-3 cyclin, cyclin A or cyclin D described herein control various steps in the regulation of cell division. Accordingly, inhibition of the activity of one or more of the cyclins described herein could lead to inhibition plant growth. Thus, the instant delta-1 cyclin, delta-3 cyclin, cyclin A or cyclin D could be appropriate for new herbicide discovery and design.

All or a substantial portion of the nucleic acid fragments of the instant invention may also be used as probes for genetically and physically mapping the genes that they are a part of, and as markers for traits linked to those genes. Such information may be useful in plant breeding in order to develop lines with desired phenotypes. For example, the instant nucleic acid fragments may be used as restriction fragment length polymorphism (RFLP) markers. Southern blots (Maniatis) of restriction-digested plant genomic DNA may be probed with the nucleic acid fragments of the instant invention. The resulting banding patterns may then be subjected to genetic analyses using computer programs such as MapMaker (Lander et at., (1987) *Genomics 1*:174-181) in order to construct a genetic map. In addition, the nucleic acid fragments of the instant invention may be used to probe Southern blots containing restriction endonuclease-treated genomic DNAs of a set of individuals representing parent and progeny of a defined genetic cross. Segregation of the DNA polymorphisms is noted and used to calculate the position of the instant nucleic acid sequence in the genetic map previously obtained using this population (Botstein, D. et al., (1980) *Am. J. Hum. Genet. 32*:314-331).

The production and use of plant gene-derived probes for use in genetic mapping is described in R. Bernatzky, R. and Tanksley, S. D. (1986) *Plant Mol. Biol. Reporter 4(1):*37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

Nucleic acid probes derived from the instant nucleic acid sequences may also be used for physical mapping (i.e., placement of sequences on physical maps; *see* Hoheisel, J. D., et al., In: *Nonmammalian Genomic Analysis: A Practical Guide,* Academic press 1996, pp. 319-346, and references cited therein).

In another embodiment, nucleic acid probes derived from the instant nucleic acid sequences may be used in direct fluorescence *in situ* hybridization (FISH) mapping (Trask, B. J. (1991) *Trends Genet. 7*:149-154). Although current methods of FISH mapping favor use of large clones (several to several hundred KB; see Laan, M. et al. (1995) *Genome Research 5*:13-20), improvements in sensitivity may allow performance of FISH mapping using shorter probes.

A variety of nucleic acid amplification-based methods of genetic and physical mapping may be carried out using the instant nucleic acid sequences. Examples include allele-specific amplification (Kazazian, H. H. (1989) *J. Lab. Clin. Med. 114(2)*:95-96), polymorphism of PCR-amplified fragments (CAPS; Sheffield, V. C. et al. (1993) *Genomics 16*:325-332), allele-specific ligation (Landegren, U. et al. (1988) *Science 241*:1077-1080), nucleotide extension reactions (Sokolov, B. P. (1990) *Nucleic Acid Res. 18*:3671), Radiation Hybrid Mapping (Walter, M. A. et al. (1997) *Nature Genetics 7*:22-28) and Happy Mapping (Dear, P. H. and Cook, P. R. (1989) *Nucleic Acid Res. 17*:6795-6807). For these methods, the sequence of a nucleic acid fragment is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

Loss of function mutant phenotypes may be identified for the instant cDNA clones either by targeted gene disruption protocols or by identifying specific mutants for these genes contained in a maize population carrying mutations in all possible genes (Ballinger and Benzer, (1989) *Proc. Natl. Acad. Sci USA 86*:9402; Koes et al., (1995) *Proc. Natl. Acad. Sci USA 92*:8149; Bensen et al., (1995) *Plant Cell 7*:75). The latter approach may be accomplished in two ways. First, short segments of the instant nucleic acid fragments may be used in polymerase chain reaction protocols in conjunction with a mutation tag sequence primer on DNAs prepared from a population of plants in which Mutator transposons or some other mutation-causing DNA element has been introduced (see Bensen, *supra*). The amplification of a specific DNA fragment with these primers indicates the insertion of the mutation tag element in or near the plant gene encoding the cyclin A, cyclin delta-1, cyclin delta-2 or cyclin delta-3. Alternatively, the instant nucleic acid fragment may be used as a hybridization probe against PCR amplification products generated from the mutation population using the mutation tag sequence primer in conjunction with an arbitrary genomic site primer, such as that for a restriction enzyme site-anchored synthetic adaptor. With either method, a plant containing a mutation in the endogenous gene encoding a cyclin A, cyclin delta-1, cyciin delta-2 or cyclin delta-3 can be identified and obtained. This mutant plant can then be used to determine or confirm the natural function of the cyclin A, cyclin delta-1, cyclin delta-2 or cyclin delta-3 gene product.

### EXAMPLES

The present invention is further defined in the following Examples, in which all parts and percentages are by weight and degrees are Celsius, unless otherwise stated. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various usages and conditions.

### EXAMPLE 1

### Composition of cDNA Libraries; Isolation and Sequencing of cDNA Clones

cDNA libraries representing mRNAs from various corn, rice, soybean and wheat tissues were prepared. The characteristics of the libraries are described below.

**TABLE 2**

| cDNA Libraries from Corn, Rice, Soybean and Wheat | | |
|---|---|---|
| Library | Tissue | Clone |
| ceb5 | Corn embryo 30 days after pollination | ceb5.pk0049.h5 |
| cen3n | Corn endosperm 20 days After pollination* | cen3n.pk0208.h3 |
| cr1n | Corn root from 7 day old seedlings* | cr1n.pk0185.g7 |
| p0072 | Corn mesocotyl: 14 days after planting etiolated seedling | p0072.comfl88rb |
| p0098 | Corn ear shoot, prophasei (2.8-4.8cm)* | p0098.cdfae90r |
| p0116 | Corn, DAM methylase induced transgenic suspension cells** | p0116.cesaf50r |
| p0128 | Corn primary and secondary immature ear tissue pooled | p0128.cpiad46rb |
| r10n | Rice 15 day old leaf* | r10n.pk0031.e6 |
| | | r10n.pk091.m14 |
| sah1c | Soybean sprayed with Authority™ herbicide | sah1c.pk003.i7 |
| scb1c | Soybean embryogenic suspension culture | scb1c.pk002.c13 |
| se6 | Soybean embryo, 26 days after flowering | se6.pk0028.f11 |
| ses9c | Soybean embryogenic suspension | ses9c.pk002.h24 |
| sfl1 | Soybean immature flower | sfl1.pk0001.a8 |
| sr1 | Soybean root | sr1.pk0001.g5 |
| | | sr1.pk0011.d11 |
| sre | Soybean root elongation | sre.pk0035.b5 |
| srm | Soybean root meristem | srm.pk0017.h9 |
| wle1n | Wheat leaf; 7 day old etiolated seedling* | wle1n.pk0036.e2 |
| wlmk1 | Wheat seedlings 1 hr after inoculation with *Erysiphe* | wlmk1.pk0009.b7 |
| | *graminis f. sp tritici* and treatment with fungicide*** | |
| wr1 | Wheat root; 7 day old seedling, light grown | wr1.pk0093.f11 |
| | | wr1.pk0112.b2 |
| wre1n | Wheat root; 7 day old etiolated seedling* | wre1n.pk0104.c1 |

| | | |
|---|---|---|
| *These libraries were normalized essentially as described in U.S. Pat. No. 5,482,845 | | |
| **cell line is transgenic for a vector harboring four copies of the estrogen response element (ERE) and CaMV-59 promoter driving dam methylase expression (Klein-Hitpab, L., et al., (1989) *Cell 46*:1053-1061). Expression of dam methylase was induced by 17 alpha-ethnylestradiol. Library was also normalized as described above | | |
| ***Application of 6-iodo-2-propoxy-3-propyl-4(3*H*)-quinazolinone; synthesis and methods of using this compound are described in USSN 08/545,827, incorporated herein by reference. | | |

cDNA libraries were prepared in Uni-ZAP™ XR vectors according to the manufacturer's protocol (Stratagene Cloning Systems, La Jolla, CA). Conversion of the Uni-ZAP™ XR libraries into plasmid libraries was accomplished according to the protocol provided by Stratagene. Upon conversion, cDNA inserts were contained in the plasmid vector pBluescript. cDNA inserts from randomly picked bacterial colonies containing recombinant pBluescript plasmids were amplified via polymerase chain reaction using primers specific for vector sequences flanking the inserted cDNA sequences or plasmid DNA was prepared from cultured bacterial cells. Amplified insert DNAs or plasmid DNAs were sequenced in dye-primer sequencing reactions to generate partial cDNA sequences (expressed sequence tags or "ESTs"; see Adams, M. D. et al., (1991) *Science 252*:1651). The resulting ESTs were analyzed using a Perkin Elmer Model 377 fluorescent sequencer.

### EXAMPLE 2

### Identification of cDNA Clones

ESTs encoding cyclin proteins were identified by conducting BLAST (Basic Local Alignment Search Tool; Altschul, S. F., et al., (1993) *J. Mol. Biol. 215*:403-410; see also www.ncbi.nlm.nih.gov/BLAST/) searches for similarity to sequences contained in the BLAST "nr" database (comprising all non-redundant GenBank CDS translations, sequences derived from the 3-dimensional structure Brookhaven Protein Data Bank, the last major release of the SWISS-PROT protein sequence database, EMBL, and DDBJ databases). The cDNA sequences obtained in Example 1 were analyzed for similarity to all publicly available DNA sequences contained in the "nr" database using the BLASTN algorithm provided by the National Center for Biotechnology Information (NCBI). The DNA sequences were translated in all reading frames and compared for similarity to all publicly available protein sequences contained in the "nr" database using the BLASTX algorithm (Gish, W. and States, D. J. (1993) *Nature Genetics 3*:266-272 and Altschul, Stephen F., et al. (1997) *Nucleic Acids Res. 25*:3389-3402) provided by the NCBI. For convenience, the P-value (probability) of observing a match of a cDNA sequence to a sequence contained in the searched databases merely by chance as calculated by BLAST are reported herein as "pLog" values, which represent the negative of the logarithm of the reported P-value. Accordingly, the greater the pLog value, the greater the likelihood that the cDNA sequence and the BLAST "hit" represent homologous proteins.

### EXAMPLE 3

### Characterization of cDNA Clones Encoding Cyclin A Proteins

The BLASTX search using the EST sequences from clones cen3n.pk0208.h3 and p0072.comfl88rb revealed similarity of the proteins encoded by the cDNAs to cyclin A from *Catharanthus roseus* (NCBI Identifier No. gi 2190259). The BLASTX search using the EST sequences from clones srm.pk0017.h9 revealed similarity of the protein encoded by the cDNA to cyclin A from *Glycine max* (NCBI Identifier No. gi 857393). The BLASTX search using the EST sequences from clones wlmk1.pk0009.b7 and wr1.pk0093.f11 revealed similarity of the proteins encoded by the cDNAs to cyclin A from *Glycine max* (NCBI Identifier No. gi 857395).

In the process of comparing the ESTs it was found that corn clones cen3n.pk0208.h3 and p0072.comfl88rb had overlapping regions of homology. Wheat clones wlmkl.pk0009.b7 and wrl.pk0093.fl1 were also found to have overlapping regions of homology. Using this homology it was possible to align the ESTs and assemble two individule contigs encoding unique corn and wheat cyclin A proteins.

The BLAST results for each of the contigs and the soybean EST are shown in Table 3:

**TABLE 3**

| BLAST Results for Clones Encoding Polypeptides Homologous to *Catharanthus roseus* and *Glycine max* Cyclin A Proteins | |
|---|---|
| Clone | BLAST pLog Score |
| Contig Composed of: cen3n.pk0208.h3 p0072.comfl88rb | 124.00 |
| srm.pk0017.h9 | 29.22 |
| Contig Composed of: wlmk1.pk0009.b7 wr1.pk0093.f11 | 72.04 |

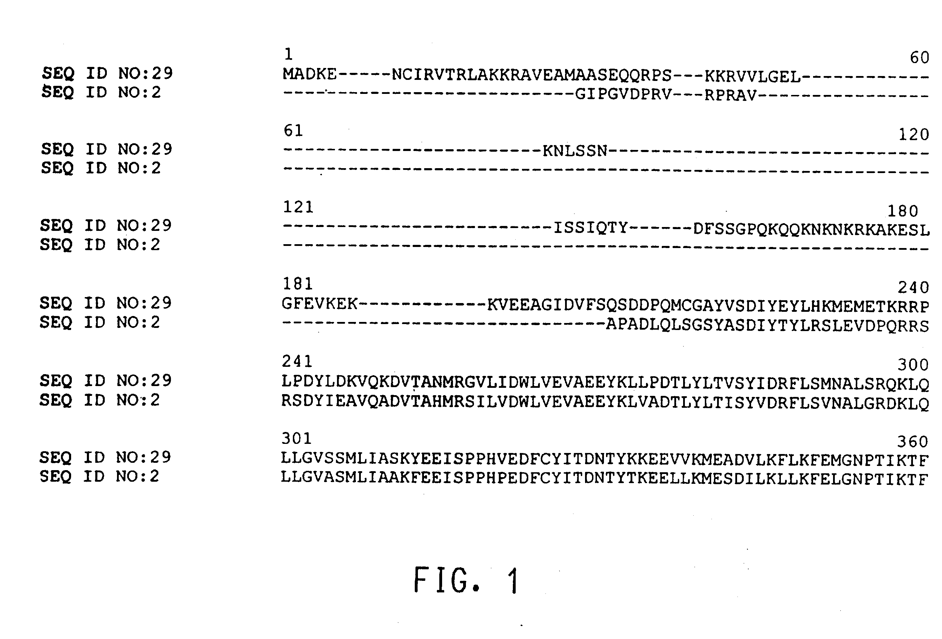

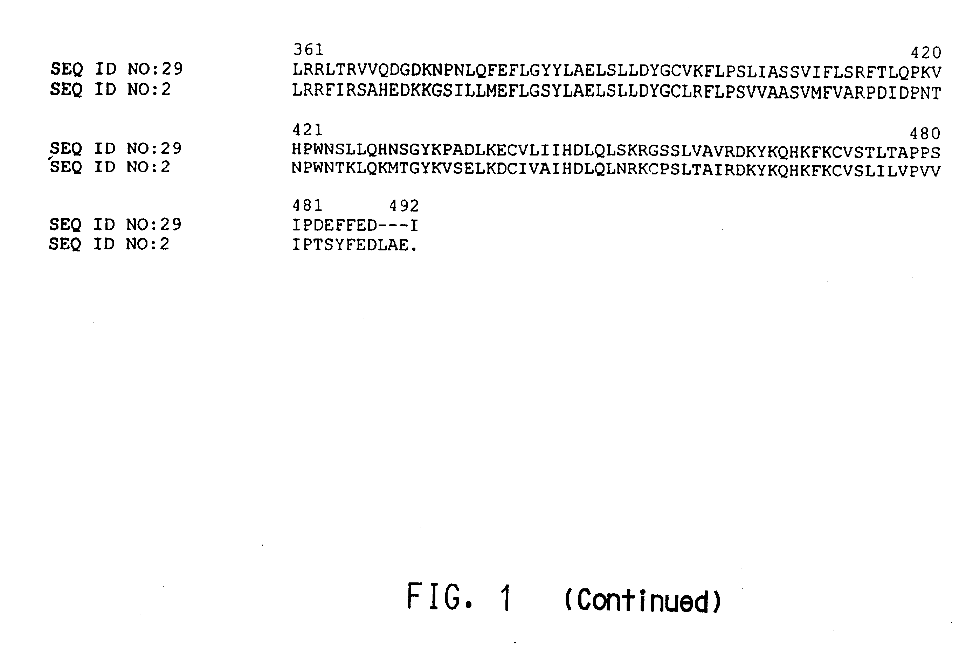

The sequence of the corn contig composed of clones cen3n.pk0208.h3 and p0072.comfl88rb is shown in SEQ ID NO:1; the deduced amino acid sequence of this contig, which represents 80% of the protein (the C-terminal region), is shown in SEQ ID NO:2. The amino acid sequence set forth in SEQ ID NO:2 was evaluated by BLASTP, yielding a pLog value of 105.00 versus the *Catharanthus roseus* sequence. Figure 1 presents an alignment of the amino acid sequence set forth in SEQ ID NO:2 and the *Catharanthus roseus* sequence. A calculation of the percent similarity of the amino acid sequence set forth in SEQ ID NO:2 and the *Catharanthus roseus* sequence revealed that SEQ ID NO:2 was 60% similar to the *Catharanthus roseus* sequence. Sequence alignments and percent similarity calculations were performed by the Clustal Algorithm (Higgins, D. G. et al., (1989) *CABIOS 5(2)*:151-153), using the Megalign program of the LASARGENE bioinformatics computing suite (DNASTAR Inc., Madison, WI) (hereinafter "Clustal Algorithm"). Default parameters for the Clustal method for protein multiple alignments were: GAP PENALTY=10, GAP LENGTH PENALTY=10; for pairwise alignments KTUPLE 1, GAP PENALTY=3, WINDOW=5 and DIAGONALS SAVED=5.

The sequence of a portion of the cDNA insert from clone srm.pk0017.h9 is shown in SEQ ID NO:3; the deduced amino acid sequence of this cDNA, which represents 22% of the protein (the N-terminal region), is shown in SEQ ID NO:4. The amino acid sequence of clone srm.pk0017.h9 appears to represent a new soybean cyclin A protein due to the fact that it was only 16% similar (as calculated by the Clustal Algorithm) to a cyclin A from *Glycine max* (NCBI Identifier No. gi 857393).

The sequence of the wheat contig composed of clones wlmk1.pk0009.b7 and wr1.pk0093.f11 is shown in SEQ ID NO:5; the deduced amino acid sequence of this contig, which represents 42% of the protein (the C-terminal region), is shown in SEQ ID NO:6. A calculation of the percent similarity of the amino acid sequence set forth in SEQ ID NO:6 and the *Glycine max* (NCBI Identifier No. gi 857395) sequence (using the Clustal Algorithm) revealed that SEQ ID NO:6 was 54% similar to the soybean cyclin A sequence.

BLAST scores and probabilities indicate that the instant nucleic acid fragments encode portions of cyclin A proteins. These sequences represent the first corn, rice and wheat sequences and a new soybean sequence encoding cyclin A proteins.

### EXAMPLE 4

### Characterization of cDNA Clones Encoding Cyclin Delta-1 Proteins

The BLASTX search using the nucleotide sequences from clones p0098.cdfae90r, p0116.cesaf50r, p0128.cpiad46rb, r10n.pk0031.e6, sah1c.pk003.i7, sr1.pk0001.g5 and se6.pk0028.f11 revealed similarity of the proteins encoded by the cDNAs to cyclin delta-1 from *Arabidopsis thaliana* (NCBI Identifier No. gi 3915635). The BLASTX search using the nucleotide sequences from clone wle1n.pk0036.e2 revealed similarity of the protein encoded by the cDNA to cyclin la from *Zea mays* (NCBI Identifier No. gi 2130119).

In the process of comparing the ESTs it was found that corn clones p0098.cdfae90r, p0116.cesaf50r and p0128.cpiad46rb had overlapping regions of homology. Soybean clones sah1c.pk003.i7 and sr1.pk0001.g5 were also found to have overlapping regions of homology. Using this homology it was possible to align the ESTs and assemble two individule contigs encoding unique corn and soybean cyclin delta-1 proteins.

The BLAST results for each of the contigs and other ESTs are shown in Table 4:

**TABLE 4**

| BLAST Results for Clones Encoding Polypeptides Homologous to *Arabidopsis thaliana* and *Zea mays* Cyclin Delta-1 Proteins | |
|---|---|
| Clone | BLAST pLog Score |
| Contig Composed of: p0098.cdfae90r p0116.cesaf50r p0128.cpiad46rb | 33.22 |
| r10n.pk0031.e6 | 22.40 |
| Contig Composed of: sah1c.pk003.i7 sr1.pk0001.g5 | 115.00 |
| se6.pk0028.f11 | 112.00 |
| wle1n.pk0036.e2 | 30.70 |

The sequence of the corn contig composed of clones p0098.cdfae90r, p0116.cesaf50r and p0128.cpiad46rb is shown in SEQ ID NO:7; the deduced amino acid sequence of this contig, which represents 71% of the protein (the N-terminal region), is shown in SEQ ID NO:8. The amino acid sequence set forth in SEQ ID NO:8 was evaluated by BLASTP, yielding a pLog value of 30.52 versus the *A. thaliana* sequence. SEQ ID NO:8 is only 18% similar (as calculated by the Clustal Algorithm) to cyclin delta-1 from *Zea mays* (NCBI Identifier No. gi 2130119) which suggests that SEQ ID NO:8 represents a new corn cyclin delta-1 protein.

A portion of the sequence of the cDNA insert in clone r10n.pk0031.e6 was determined and is shown in SEQ ID NO:9; the deduced amino acid sequence of this cDNA, which represents 53% of the protein (the N-terminal region), is shown in SEQ ID NO:10. The amino acid sequence set forth in SEQ ID NO:10 was evaluated by BLASTP, yielding a pLog value of 22.00 versus the *A. thaliana* sequence.

The sequence of the soybean contig composed of clones sah1c.pk003.i7 and sr1.pk0001.g5 is shown in SEQ ID NO: 11; the deduced amino acid sequence of this contig, which represents 100% of the protein, is shown in SEQ ID NO:12. The amino acid sequence set forth in SEQ ID NO:12 was evaluated by BLASTP, yielding a pLog value of 108.00 versus the *A. thaliana* sequence.

The entire sequence of the cDNA insert in clone se6.pk0028.f11 was determined and is shown in SEQ ID NO:13; the deduced amino acid sequence of this cDNA, which represents 95% of the protein, is shown in SEQ ID NO:14. The amino acid sequence set forth in SEQ ID NO:14 was evaluated by BLASTP, yielding a pLog value of 103.00 versus the *A. thaliana* sequence.

The sequence of a portion of the cDNA insert from clone wle1n.pk0036.e2 is shown in SEQ ID NO:15; the deduced amino acid sequence of this cDNA is shown in SEQ ID NO:16.

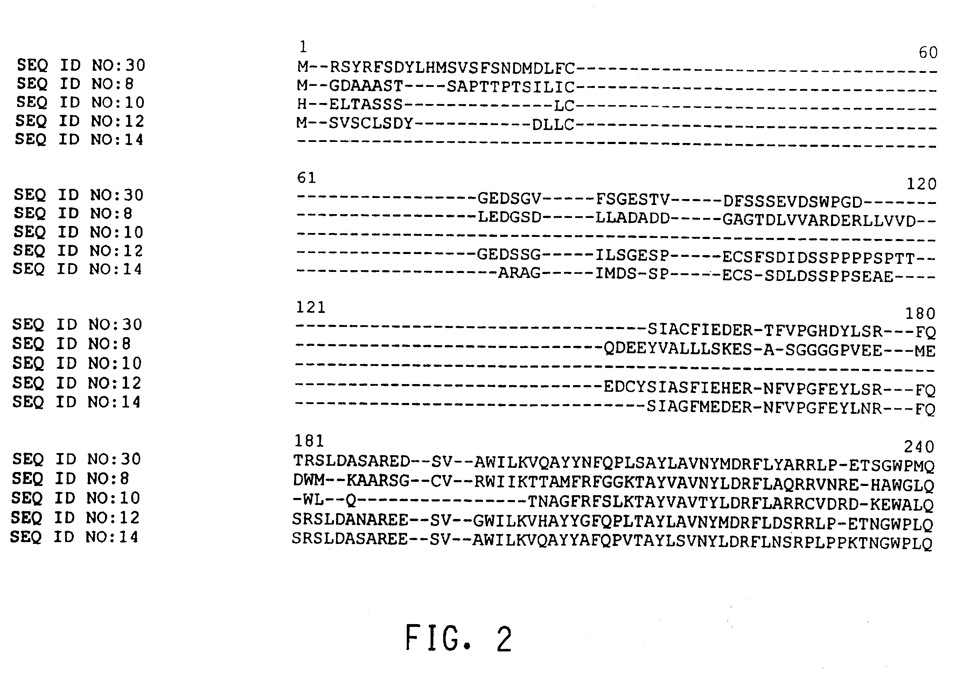

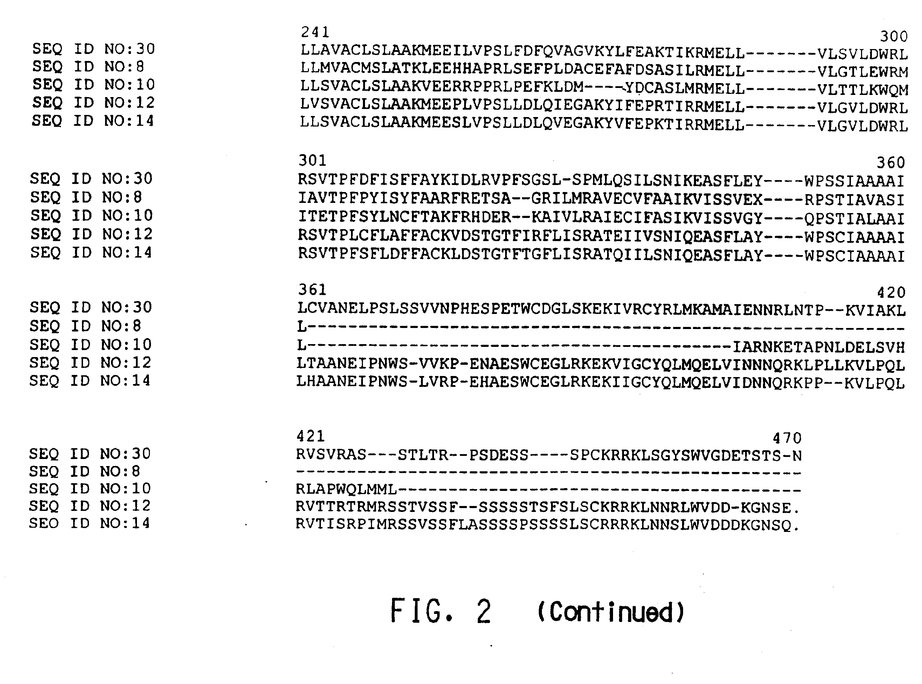

Figure 2 presents an alignment of the amino acid sequences set forth in SEQ ID NOs:8, 10,12 and 14 and the *A. thaliana* cyclin delta-1 sequence. The data in Table 5 represents a calculation of the percent similarity of the amino acid sequences set forth in SEQ ID NOs:8, 10, 12 and 14 and the *A. thaliana* cyclin delta-1 sequence.

**TABLE 5**

| Percent Identity of Amino Acid Sequences Deduced From the Nucleotide Sequences of cDNA Clones Encoding Polypeptides Homologous to *Arabidopsis thaliana* Cyclin Delta-1 Proteins | | |
|---|---|---|
| Clone | SEQ ID NO. | Percent Similarity |
| Contig Composed of: p0098.cdfae90r p0116.cesaf50r p0128.cpiad46rb | 8 | 29% |
| r10n.pk0031.e6 | 10 | 31% |
| Contig Composed of: sah1c.pk003.i7 sr1.pk0001.g5 | 12 | 54% |
| se6.pk0028.f11 | 14 | 54% |

Sequence alignments and sequence percent similarity calculations were performed by the Clustal Algorithm. Sequence alignments, BLAST scores and probabilities indicate that the instant nucleic acid fragments encode entire, nearly entire or portions of cyclin delta-1 proteins. These sequences represent the first rice, soybean and wheat sequences and a new corn sequence encoding cyclin delta-1 proteins.

### EXAMPLE 5

### Characterization of cDNA Clones Encoding Cyclin Delta-2 Proteins

The BLASTX search using the nucleotide sequences from clones ceb5.pk0049.h5, r10n.pk091.m14, and wre1n.pk0104.c1 revealed similarity of the proteins encoded by the cDNAs to cyclin delta-2 from *Nicotina tabacum* (NCBI Identifier No. gi 4160298). The BLAST results for each of these ESTs are shown in Table 6:

**TABLE 6**

| BLAST Results for Clones Encoding Polypeptides Homologous to *Nicotina tabacum* Cyclin Delta-2 Proteins | |
|---|---|
| Clone | BLAST pLog Score |
| ceb5.pk0049.h5 | 65.22 |
| r10n.pk091.m14 | 9.10 |
| wre1n.pk0104.c1 | 19.70 |

The sequence of the entire cDNA insert in clone ceb5.pk0049.h5 was determined and is shown in SEQ ID NO:17; the deduced amino acid sequence of this cDNA, which represents 94% of the protein, is shown in SEQ ID NO:18. The amino acid sequence set forth in SEQ ID NO:18 was evaluated by BLASTP, yielding a pLog value of 64.10 versus the *Nicotina tabacum* sequence.

The sequence of a portion of the cDNA insert from clone r10n.pk091.m14 is shown in SEQ ID NO:19; the deduced amino acid sequence of this cDNA, which represents 30% of a cyclin delta-2 protein (the C-terminal region), is shown in SEQ ID NO:20.

The sequence of the entire cDNA insert in clone wre1n.pk0104.c1 was determined and is shown in SEQ ID NO:21; the deduced amino acid sequence of this cDNA, which represents 26% of the protein (the C-terminal region), is shown in SEQ ID NO:22. The amino acid sequence set forth in SEQ ID NO:22 was evaluated by BLASTP, yielding a pLog value of 13.00 versus the *Nicotina tabacum* sequence.

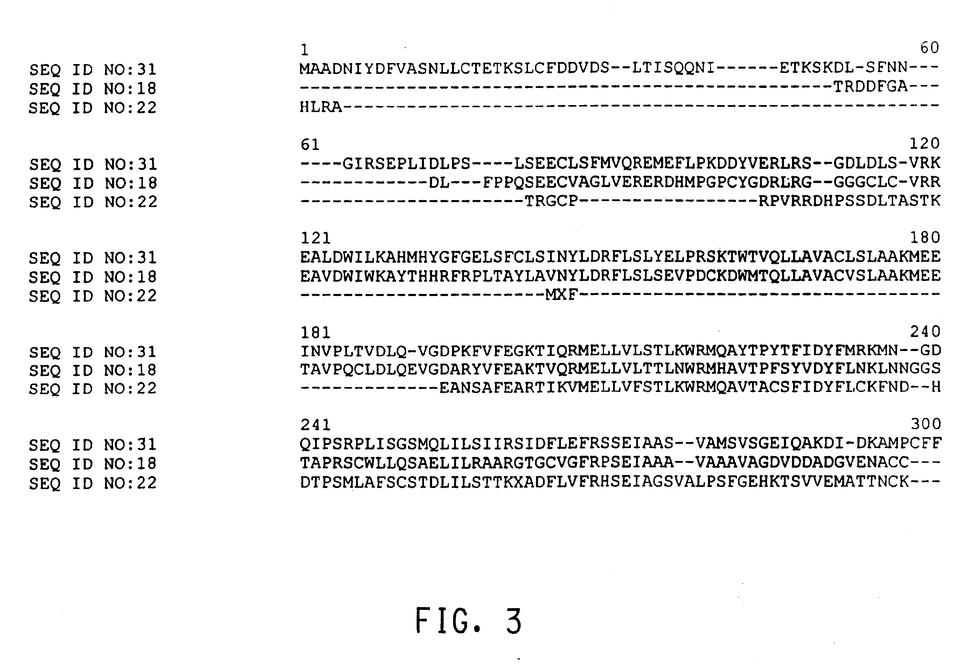

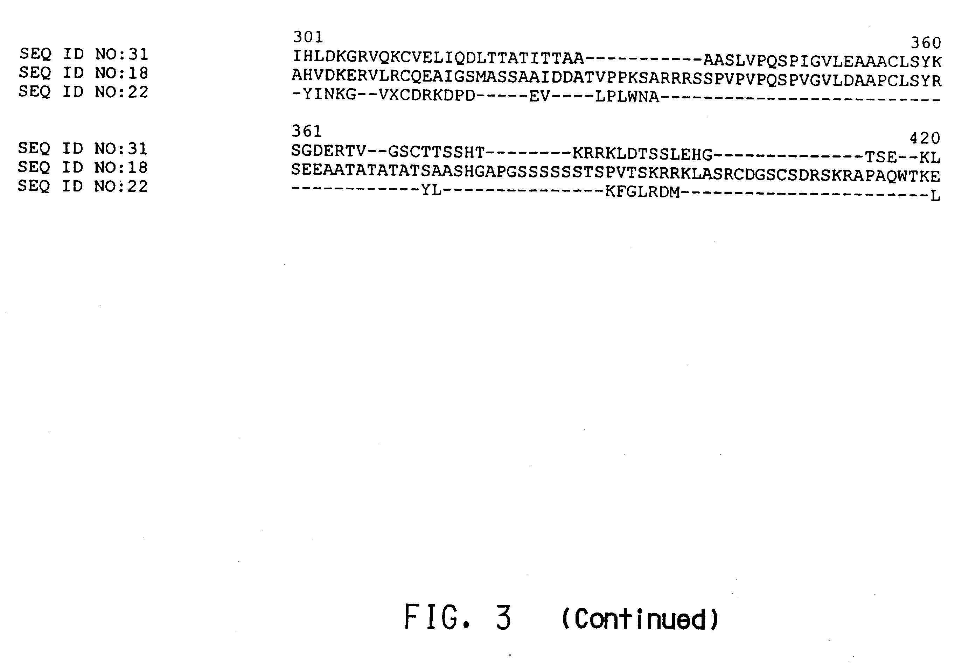

Figure 3 presents an alignment of the amino acid sequences set forth in SEQ ID NOs:18 and 22 and the *Nicotina tabacum* sequence. The data in Table 7 represents a calculation of the percent similarity of the amino acid sequences set forth in SEQ ID NOs:18 22 and the *Nicotina tabacum* sequence.

**TABLE 7**

| Percent Identity of Amino Acid Sequences Deduced From the Nucleotide Sequences of cDNA Clones Encoding Polypeptides Homologous to *Nicotina tabacum* Cyclin Delta-2 Proteins | | |
|---|---|---|
| Clone | SEQ ID NO. | Percent Similarity |
| ceb5.pk0049.h5 | 18 | 37% |
| wre1n.pk0104.c1 | 22 | 32.5% |

Sequence alignments and sequence percent similarity calculations were performed by the Clustal Algorithm. Sequence alignments and BLAST scores and probabilities indicate that the instant nucleic acid fragments encode entire, nearly entire or portions of cyclin delta-2 proteins. These sequences represent the first corn, rice and wheat sequences encoding cyclin delta-2 proteins.

### EXAMPLE 6

### Characterization of cDNA Clones Encoding Cyclin Delta-3 Proteins

The BLASTX search using the nucleotide sequences from clones cr1n.pk0185.g7, ses9c.pk002.h24, scb1c.pk002.c13 and sr1.pk0011.d11 revealed similarity of the proteins encoded by the cDNAs to cyclin delta-3 from *Nicotiana tabacum* (NCBI Identifier No. gi 4160300). The BLASTX search using the nucleotide sequences from clones sfl1.pk0001.a8 and sre.pk0035.b5 revealed similarity of the proteins encoded by the cDNAs to cyclin delta-3 from *Pisum sativum* (NCBI Identifier No. gi 3608179).

In the process of comparing the ESTs it was found that soybean clones ses9c.pk002.h24, scb1c.pk002.c13 and sr1.pk0011.d11 had overlapping regions of homology. Soybean clones sfl1.pk0001.a8 and sre.pk0035.b5 were also found to have overlapping regions of homology. Using this homology it was possible to align the ESTs and assemble two contigs encoding unique soybean cyclin delta-3 proteins.

The BLAST results for each of these contigs and the corn EST are shown in Table 8:

**TABLE 8**

| BLAST Results for Clones Encoding Polypeptides Homologous to *Nicotiana tabacum* and *Pisum sativum* Cyclin Delta-3 Proteins | |
|---|---|
| Clone | BLAST pLog Score |
| cr1n.pk0185.g7 | 35.30 |
| Contig Composed of: ses9c.pk002.h24 scb1c.pk002.c13 sr1.pk0011.d11 | 29.22 |
| Contig Composed of: sfl1.pk0001.a8 sre.pk0035.b5 | 18.00 |

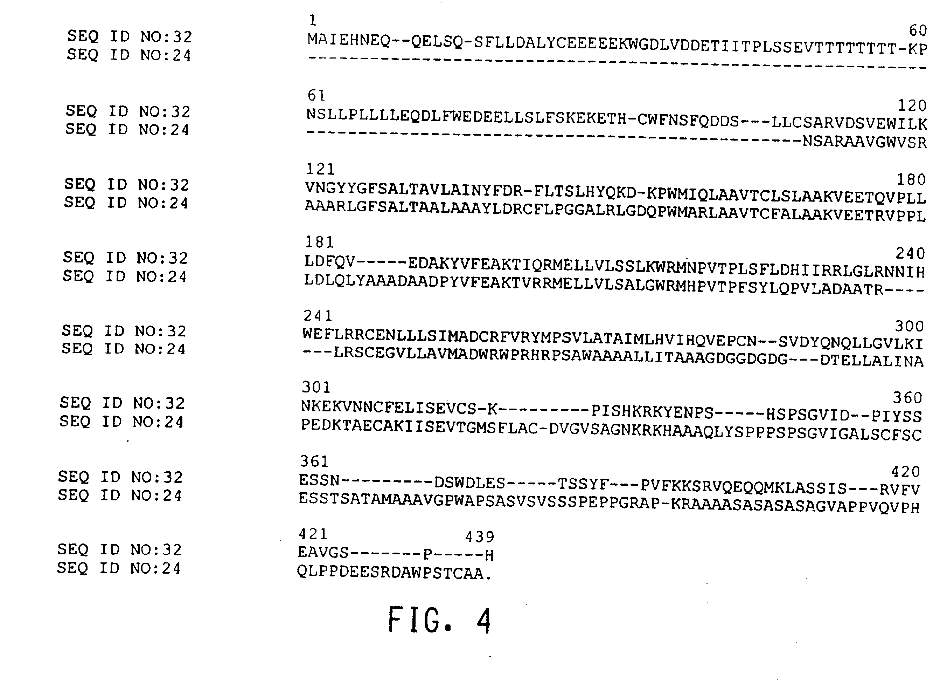

A large portion of the cDNA insert in clone crln.pk0185.g7 was determined and is shown in SEQ ID NO:23; the deduced amino acid sequence of this cDNA, which represents 85% of the protein (C-terminal region) is shown in SEQ ID NO:24. The amino acid sequence set forth in SEQ ID NO:24 was evaluated by BLASTP, yielding a pLog value of 38.10 versus the *Nicotiana tabacum* sequence. A calculation (using the Clustal Algorithm) of the percent similarity of the amino acid sequence set forth in SEQ ID NO:24 and the *Nicotiana tabacum* sequence revealed SEQ ID NO:24 was 27% to the *Nicotiana tabacum* sequence. Figure 4 presents an alignment of the amino acid sequences set forth in SEQ ID NO:24 and the *Nicotiana tabacum* sequence.

The sequence of the soybean contig composed of clones ses9c.pk002.h24, scb1c.pk002.c13 and sr1.pk0011.d11 is shown in SEQ ID NO:25; the deduced amino acid sequence of this contig, which represents 42% of the protein (the N-terminal region) is shown in SEQ ID NO:26.

The sequence of the soybean contig composed of clones sfl1.pk0001.a8 and sre.pk0035.b5 is shown in SEQ ID NO:27; the deduced amino acid sequence of this contig, which represents 21% of the protein (N-terminal region) is shown in SEQ ID NO:28.

Sequence alignments and BLAST scores and probabilities indicate that the instant nucleic acid fragments encode entire, nearly entire or portions of cyclin delta-3 proteins. These sequences represent the first corn and soybean sequences encoding cyclin delta-3 proteins.

### EXAMPLE 7

### Expression of Chimeric Genes in Monocot Cells

A chimeric gene comprising a cDNA encoding a cyclin protein in sense orientation with respect to the maize 27 kD zein promoter that is located 5' to the cDNA fragment, and the 10 kD zein 3' end that is located 3' to the cDNA fragment, can be constructed. The cDNA fragment of this gene may be generated by polymerase chain reaction (PCR) of the cDNA clone using appropriate oligonucleotide primers. Cloning sites (NcoI or SmaI) can be incorporated into the oligonucleotides to provide proper orientation of the DNA fragment when inserted into the digested vector pML103 as described below. Amplification is then performed in a standard PCR. The amplified DNA is then digested with restriction enzymes NcoI and Smal and fractionated on an agarose gel. The appropriate band can be isolated from the gel and combined with a 4.9 kb NcoI-SmaI fragment of the plasmid pML103. Plasmid pML103 has been deposited under the terms of the Budapest Treaty at ATCC (American Type Culture Collection, 10801 University Blvd., Manassas, VA 20110-2209), and bears accession number ATCC 97366. The DNA segment from pML103 contains a 1.05 kb SalI-NcoI promoter fragment of the maize 27 kD zein gene and a 0.96 kb SmaI-SalI fragment from the 3' end of the maize 10 kD zein gene in the vector pGem9Zf(+) (Promega). Vector and insert DNA can be ligated at 15°C overnight, essentially as described (Maniatis). The ligated DNA may then be used to transform *E. coli* XL1-Blue (Epicurian Coli XL-1 Blue™; Stratagene). Bacterial transformants can be screened by restriction enzyme digestion of plasmid DNA and limited nucleotide sequence analysis using the dideoxy chain termination method (Sequenase™ DNA Sequencing Kit; U. S. Biochemical). The resulting plasmid construct would comprise a chimeric gene encoding, in the 5' to 3' direction, the maize 27 kD zein promoter, a cDNA fragment encoding a cyclin protein, and the 10 kD zein 3' region.

The chimeric gene described above can then be introduced into corn cells by the following procedure. Immature corn embryos can be dissected from developing caryopses derived from crosses of the inbred corn lines H99 and LH132. The embryos are isolated 10 to 11 days after pollination when they are 1.0 to 1.5 mm long. The embryos are then placed with the axis-side facing down and in contact with agarose-solidified N6 medium (Chu et al., (1975) *Sci. Sin. Peking* 18:659-668). The embryos are kept in the dark at 27°C. Friable embryogenic callus consisting of undifferentiated masses of cells with somatic proembryoids and embryoids borne on suspensor structures proliferates from the scutellum of these immature embryos. The embryogenic callus isolated from the primary explant can be cultured on N6 medium and sub-cultured on this medium every 2 to 3 weeks.

The plasmid, p35S/Ac (obtained from Dr. Peter Eckes, Hoechst Ag, Frankfurt, Germany) may be used in transformation experiments in order to provide for a selectable marker. This plasmid contains the *Pat* gene (see European Patent Publication 0 242 236) which encodes phosphinothricin acetyl transferase (PAT). The enzyme PAT confers resistance to herbicidal glutamine synthetase inhibitors such as phosphinothricin. The *pat* gene in p35S/Ac is under the control of the 35S promoter from Cauliflower Mosaic Virus (Odell et al. (1985) *Nature* 313:810-812) and the 3' region of the nopaline synthase gene from the T-DNA of the Ti plasmid of *Agrobacterium tumefaciens*.

The particle bombardment method (Klein et al., (1987) *Nature* 327:70-73) may be used to transfer genes to the callus culture cells. According to this method, gold particles (1 µm in diameter) are coated with DNA using the following technique. Ten µg of plasmid DNAs are added to 50 µL of a suspension of gold particles (60 µg per mL). Calcium chloride (50 µL of a 2.5 M solution) and spermidine free base (20 µL of a 1.0 M solution) are added to the particles. The suspension is vortexed during the addition of these solutions. After 10 minutes, the tubes are briefly centrifuged (5 sec at 15,000 rpm) and the supernatant removed. The particles are resuspended in 200 µL of absolute ethanol, centrifuged again and the supernatant removed. The ethanol rinse is performed again and the particles resuspended in a final volume of 30 µL of ethanol. An aliquot (5 µL) of the DNA-coated gold particles can be placed in the center of a Kapton™ flying disc (Bio-Rad Labs). The particles are then accelerated into the corn tissue with a Biolistic™ PDS-1000/He (Bio-Rad Instruments, Hercules CA), using a helium pressure of 1000 psi, a gap distance of 0.5 cm and a flying distance of 1.0 cm.

For bombardment, the embryogenic tissue is placed on filter paper over agarose-solidified N6 medium. The tissue is arranged as a thin lawn and covered a circular area of about 5 cm in diameter. The petri dish containing the tissue can be placed in the chamber of the PDS-1000/He approximately 8 cm from the stopping screen. The air in the chamber is then evacuated to a vacuum of 28 inches of Hg. The macrocarrier is accelerated with a helium shock wave using a rupture membrane that bursts when the He pressure in the shock tube reaches 1000 psi.

Seven days after bombardment the tissue can be transferred to N6 medium that contains gluphosinate (2 mg per liter) and lacks casein or proline. The tissue continues to grow slowly on this medium. After an additional 2 weeks the tissue can be transferred to fresh N6 medium containing gluphosinate. After 6 weeks, areas of about 1 cm in diameter of actively growing callus can be identified on some of the plates containing the glufosinate-supplemented medium. These calli may continue to grow when sub-cultured on the selective medium.

Plants can be regenerated from the transgenic callus by first transferring clusters of tissue to N6 medium supplemented with 0.2 mg per liter of 2,4-D. After two weeks the tissue can be transferred to regeneration medium (Fromm et al., (1990) *Bio*/*Technology 8*:833-839).

### EXAMPLE 8

### Expression of Chimeric Genes in Dicot Cells

A seed-specific expression cassette composed of the promoter and transcription terminator from the gene encoding the β subunit of the seed storage protein phaseolin from the bean *Phaseolus vulgaris* (Doyle et al. (1986) *J. Biol. Chem. 261*:9228-9238) can be used for expression of the instant cyclin proteins in transformed soybean. The phaseolin cassette includes about 500 nucleotides upstream (5') from the translation initiation codon and about 1650 nucleotides downstream (3') from the translation stop codon of phaseolin. Between the 5' and 3' regions are the unique restriction endonuclease sites Nco I (which includes the ATG translation initiation codon), Sma I, Kpn I and Xba I. The entire cassette is flanked by Hind III sites.

The cDNA fragment of this gene may be generated by polymerase chain reaction (PCR) of the cDNA clone using appropriate oligonucleotide primers. Cloning sites can be incorporated into the oligonucleotides to provide proper orientation of the DNA fragment when inserted into the expression vector. Amplification is then performed as described above, and the isolated fragment is inserted into a pUC18 vector carrying the seed expression cassette.

Soybean embroys may then be transformed with the expression vector comprising sequences encoding cyclin proteins. To induce somatic embryos, cotyledons, 3-5 mm in length dissected from surface sterilized, immature seeds of the soybean cultivar A2872, can be cultured in the light or dark at 26°C on an appropriate agar medium for 6-10 weeks. Somatic embryos which produce secondary embryos are then excised and placed into a suitable liquid medium. After repeated selection for clusters of somatic embryos which multiplied as early, globular staged embryos, the suspensions are maintained as described below.

Soybean embryogenic suspension cultures can maintained in 35 mL liquid media on a rotary shaker, 150 rpm, at 26°C with florescent lights on a 16:8 hour day/night schedule. Cultures are subcultured every two weeks by inoculating approximately 35 mg of tissue into 35 mL of liquid medium.

Soybean embryogenic suspension cultures may then be transformed by the method of particle gun bombardment (Kline et al. (1987) *Nature* (London) *327*:70, U.S. Patent No. 4,945,050). A DuPont Biolistic™ PDS1000/HE instrument (helium retrofit) can be used for these transformations.

A selectable marker gene which can be used to facilitate soybean transformation is a chimeric gene composed of the 35S promoter from Cauliflower Mosaic Virus (Odell et al. (1985) *Nature 313*:810-812), the hygromycin phosphotransferase gene from plasmid pJR225 (from *E. coli;* Gritz et al.(1983) *Gene 25*:179-188) and the 3' region of the nopaline synthase gene from the T-DNA of the Ti plasmid of *Agrobacterium tumefaciens*. The seed expression cassette comprising the phaseolin 5' region, the fragment encoding the cyclin protein and the phaseolin 3' region can be isolated as a restriction fragment. This fragment can then be inserted into a unique restriction site of the vector carrying the marker gene.

To 50 µL of a 60 mg/mL 1 µm gold particle suspension is added (in order): 5 µL DNA (1 µg/µL), 20 µl spermidine (0.1 M), and 50 µL CaCl₂ (2.5 M). The particle preparation is then agitated for three minutes, spun in a microfuge for 10 seconds and the supernatant removed. The DNA-coated particles are then washed once in 400 µL 70% ethanol and resuspended in 40 µL of anhydrous ethanol. The DNA/particle suspension can be sonicated three times for one second each. Five µL of the DNA-coated gold particles are then loaded on each macro carrier disk.

Approximately 300-400 mg of a two-week-old suspension culture is placed in an empty 60x15 mm petri dish and the residual liquid removed from the tissue with a pipette. For each transformation experiment, approximately 5-10 plates of tissue are normally bombarded. Membrane rupture pressure is set at 1100 psi and the chamber is evacuated to a vacuum of 28 inches mercury. The tissue is placed approximately 3.5 inches away from the retaining screen and bombarded three times. Following bombardment, the tissue can be divided in half and placed back into liquid and cultured as described above.

Five to seven days post bombardment, the liquid media may be exchanged with fresh media, and eleven to twelve days post bombardment with fresh media containing 50 mg/mL hygromycin. This selective media can be refreshed weekly. Seven to eight weeks post bombardment, green, transformed tissue may be observed growing from untransformed, necrotic embryogenic clusters. Isolated green tissue is removed and inoculated into individual flasks to generate new, clonally propagated, transformed embryogenic suspension cultures. Each new line may be treated as an independent transformation event. These suspensions can then be subcultured and maintained as clusters of immature embryos or regenerated into whole plants by maturation and germination of individual somatic embryos.

### EXAMPLE 9

### Expression of Chimeric Genes in Microbial Cells

The cDNAs encoding the instant cyclin proteins can be inserted into the T7 *E*. *coli* expression vector pBT430. This vector is a derivative of pET-3a (Rosenberg et al. (1987) *Gene 56*:125-135) which employs the bacteriophage T7 RNA polymerase/T7 promoter system. Plasmid pBT430 was constructed by first destroying the EcoR I and Hind III sites in pET-3a at their original positions. An oligonucleotide adaptor containing EcoR I and Hind III sites was inserted at the BamH I site of pET-3a. This created pET-3aM with additional unique cloning sites for insertion of genes into the expression vector. Then, the Nde I site at the position of translation initiation was converted to an Nco I site using oligonucleotide-directed mutagenesis. The DNA sequence of pET-3aM in this region, 5'-CATATGG, was converted to 5'-CCCATGG in pBT430.

Plasmid DNA containing a cDNA may be appropriately digested to release a nucleic acid fragment encoding the protein. This fragment may then be purified on a 1% NuSieve GTG™ low melting agarose gel (FMC). Buffer and agarose contain 10 µg/ml ethidium bromide for visualization of the DNA fragment. The fragment can then be purified from the agarose gel by digestion with GELase™ (Epicentre Technologies) according to the manufacturer's instructions, ethanol precipitated, dried and resuspended in 20 µL of water. Appropriate oligonucleotide adapters may be ligated to the fragment using T4 DNA ligase (New England Biolabs, Beverly, MA). The fragment containing the ligated adapters can be purified from the excess adapters using low melting agarose as described above. The vector pBT430 is digested, dephosphorylated with alkaline phosphatase (NEB) and deproteinized with phenol/chloroform as decribed above. The prepared vector pBT430 and fragment can then be ligated at 16°C for 15 hours followed by transformation into DH5 electrocompetent cells (GIBCO BRL). Transformants can be selected on agar plates containing LB media and 100 µg/mL ampicillin. Transformants containing the gene encoding the cyclin protein are then screened for the correct orientation with respect to the T7 promoter by restriction enzyme analysis.

For high level expression, a plasmid clone with the cDNA insert in the correct orientation relative to the T7 promoter can be transformed into *E. coli* strain BL21(DE3) (Studier et al. (1986) *J. Mol. Biol. 189*:113-130). Cultures are grown in LB medium containing ampicillin (100 mg/L) at 25°C. At an optical density at 600 nm of approximately 1, IPTG (isopropylthio-β-galactoside, the inducer) can be added to a final concentration of 0.4 mM and incubation can be continued for 3 h at 25°. Cells are then harvested by centrifugation and re-suspended in 50 µL of 50 mM Tris-HCl at pH 8.0 containing 0.1 mM DTT and 0.2 mM phenyl methylsulfonyl fluoride. A small amount of 1 mm glass beads can be added and the mixture sonicated 3 times for about 5 seconds each time with a microprobe sonicator. The mixture is centrifuged and the protein concentration of the supernatant determined. One µg of protein from the soluble fraction of the culture can be separated by SDS-polyacrylamide gel electrophoresis. Gels can be observed for protein bands migrating at the expected molecular weight.

### EXAMPLE 10

### Evaluating Compounds for Their Ability to Inhibit the Activity of Cyclin Proteins

The cyclin proteins described herein may be produced using any number of methods known to those skilled in the art. Such methods include, but are not limited to, expression in bacteria as described in Example 9, or expression in eukaryotic cell culture, *in planta*, and using viral expression systems in suitably infected organisms or cell lines. The instant cyclin proteins may be expressed either as mature forms of the proteins as observed *in vivo* or as fusion proteins by covalent attachment to a variety of enzymes, proteins or affinity tags. Common fusion protein partners include glutathione S-transferase ("GST"), thioredoxin ("Trx"), maltose binding protein, and C- and/or N-terminal hexahistidine polypeptide ("(His)₆"). The fusion proteins may be engineered with a protease recognition site at the fusion point so that fusion partners can be separated by protease digestion to yield intact mature enzyme. Examples of such proteases include thrombin, enterokinase and factor Xa. However, any protease can be used which specifically cleaves the peptide connecting the fusion protein and the enzyme.

Purification of the instant cyclin proteins, if desired, may utilize any number of separation technologies familiar to those skilled in the art of protein purification. Examples of such methods include, but are not limited to, homogenization, filtration, centrifugation, heat denaturation, ammonium sulfate precipitation, desalting, pH precipitation, ion exchange chromatography, hydrophobic interaction chromatography and affinity chromatography, wherein the affinity ligand represents a substrate, substrate analog or inhibitor. When the cyclin proteins are expressed as fusion proteins, the purification protocol may include the use of an affinity resin which is specific for the fusion protein tag attached to the expressed enzyme or an affinity resin containing ligands which are specific for the enzyme. For example, a cyclin protein may be expressed as a fusion protein coupled to the C-terminus of thioredoxin. In addition, a (His)₆ peptide may be engineered into the N-terminus of the fused thioredoxin moiety to afford additional opportunities for affinity purification. Other suitable affinity resins could be synthesized by linking the appropriate ligands to any suitable resin such as Sepharose-4B. In an alternate embodiment, a thioredoxin fusion protein may be eluted using dithiothreitol; however, elution may be accomplished using other reagents which interact to displace the thioredoxin from the resin. These reagents include β-mercaptoethanol or other reduced thiol. The eluted fusion protein may be subjected to further purification by traditional means as stated above, if desired. Proteolytic cleavage of the thioredoxin fusion protein and the enzyme may be accomplished after the fusion protein is purified or while the protein is still bound to the ThioBond™ affinity resin or other resin.

Crude, partially purified or purified enzyme, either alone or as a fusion protein, may be utilized in assays for the evaluation of compounds for their ability to inhibit enzymatic activition of the cyclin proteins disclosed herein. Assays may be conducted under well known experimental conditions which permit optimal enzymatic activity. For example, assays for cyclin A are presented by Kouchi, H., et al., (1995) *Plant Cell 7(8)*:143-1155. Assays for cyclin delta-1 and cyclin delta-2 are presented by Soni B., et al., (1995) *Plant Cell 7(1)*:85-103. Assays for cyclin delta-3 are presented by Sorrell D. A., et al., (1999) *Plant Physiol. 199*:343-351.

## Claims

1. An isolated nucleic acid fragment encoding all or a substantial portion of a cyclin delta-2 protein comprising a member selected from the group consisting of:
(a) an isolated nucleic acid fragment encoding all or a substantial portion of the amino acid sequence set forth in
SEQ ID NO:18;
(b) an isolated nucleic acid fragment that is substantially similar to an isolated nucleic acid fragment encoding all or a substantial portion of the amino acid sequence set forth in
SEQ ID NO: 18; and
(c) an isolated nucleic acid fragment that is complementary to (a) or (b).

2. The isolated nucleic acid fragment of Claim 1 wherein the nucleotide sequence of the fragment comprises all or a portion of the sequence set forth in
SEQ ID NO: 17.

3. A chimeric gene comprising the nucleic acid fragment of Claim 1 operably linked to suitable regulatory sequences.

4. A transformed host cell comprising the chimeric gene of Claim 3.

5. A cyclin delta-2 polypeptide comprising all or a substantial portion of the amino acid sequence set forth in SEQ ID NO: 18.

6. A method of altering the level of expression of a cyclin protein in a host cell comprising:
(a) transforming a host cell with the chimeric gene of Claim 13; and
(b) growing the transformed host cell produced in step (a) under conditions that are suitable for expression of the chimeric gene
wherein expression of the chimeric gene results in production of altered levels of a cyclin protein in the transformed host cell.

7. A method of obtaining a nucleic acid fragment encoding all or a substantial portion of the amino acid sequence encoding a cyclin protein comprising:
(a) probing a cDNA or genomic library with the nucleic acid fragment of Claim 1;
(b) identifying a DNA clone that hybridizes with the nucleic acid fragment of any of Claim 1;
(c) isolating the DNA clone identified in step (b); and
(d) sequencing the cDNA or genomic fragment that comprises the clone isolated in step (c)
wherein the sequenced nucleic acid fragment encodes all or a substantial portion of the amino acid sequence encoding a cyclin protein.

8. A method of obtaining a nucleic acid fragment encoding a substantial portion of an amino acid sequence encoding a cyclin protein comprising:
(a) synthesizing an oligonucleotide primer corresponding to a portion of the sequence set forth in SEQ ID NO: 17; and
(b) amplifying a cDNA insert present in a cloning vector using the oligonucleotide primer of step (a) and a primer representing sequences of the cloning vector
wherein the amplified nucleic acid fragment encodes a substantial portion of an amino acid sequence encoding a cyclin protein.

9. The product of the method of Claim 7.

10. The product of the method of Claim 8.

11. A method for evaluating at least one compound for its ability to inhibit the activity of a cyclin protein, the method comprising the steps of:
(a) transforming a host cell with a chimeric gene comprising a nucleic acid fragment encoding a cyclin protein, operably linked to suitable regulatory sequences;
(b) growing the transformed host cell under conditions that are suitable for expression of the chimeric gene wherein expression of the chimeric gene results in production of the cyclin protein encoded by the operably linked nucleic acid fragment in the transformed host cell;
(c) optionally purifying the cyclin protein expressed by the transformed host cell;
(d) treating the cyclin protein with a compound to be tested; and
(e) comparing the activity of the cyclin protein that has been treated with a test compound to the activity of an untreated cyclin protein, thereby selecting compounds with potential for inhibitory activity.
